# EUROPEAN PATENT APPLICATION

(11) **EP 4 818 207 A1**
(43) Date of publication of application: **07.10.2026**
(21) Application number: 24898164.9
(22) Date of filing: 28.11.2024
(51) Int. Cl.: C07K 16/28, C07K 14/47, A61P 35/00, A61K 39/00

(54) **NOVEL RECOMBINANT ANTIBODY SPECIFICALLY BINDING TO B CELL ANTIGEN, AND USE THEREOF**

(30) Priority: 30.11.2023 KR 20230170574
(71) Applicant: UIF (University Industry Foundation), Yonsei University, Seoul 03722 (KR)
(72) Inventor: KIM, Joo Young, Goyang-si Gyeonggi-do 10394 (KR); LEE, Donghyuk, Seoul 05025 (KR); CHOI, Ayeon, Yongin-si Gyeonggi-do 16860 (KR)
(74) Representative: Müller, Christian Stefan Gerd
(86) International application number: PCT/KR2024/019179
(87) International publication number: WO 2025/116570

(57) **Abstract**

The present invention relates to a recombinant polypeptide that specifically binds to B lymphocytes and a composition for preventing or treating B-cell lymphoma, comprising the recombinant polypeptide as an active ingredient. The present invention can significantly enhance direct cell death activity against tumor cells by conjugating concanavalin A, a ligand of MPZL1, to an antibody molecule that recognizes a B-lymphocyte-specific antigen such as CD20. Accordingly, unlike therapeutic antibodies that act mainly through antibody-dependent cell-mediated cytotoxicity (ADCC), complement-dependent cytotoxicity (CDC) and antibody-dependent cellular phagocytosis (ADP), the recombinant polypeptide of the present invention does not depend on effector mechanisms of a patient's immune system. Therefore, the recombinant polypeptide can be usefully employed as an effective therapeutic composition even for cancer patients with reduced immune activity.

## Description

### Technical Field

The present invention relates to a novel recombinant antibody having significantly enhanced direct cell death (DCD) activity against B-cell lymphoma.

### Background Art

CD20 is a B-lymphocyte specific antigen expressed at high concentrations only on the surface of normal and tumor cells of the B-lymphocyte lineage. It functions as a receptor during B-cell activation and is a hydrophobic protein containing four transmembrane domains, with both the N-terminus and C-terminus located within the cytoplasm.

The CD20 antigen is not expressed at detectable levels in hematopoietic stem cells, B-cell progenitor cells, normal plasma cells or other normal tissues, but is expressed in more than 90% of B-cell non-Hodgkin lymphoma (NHL) cases. CD20 is predicted to be involved in the activation process for cell cycle initiation and differentiation and is predicted to function as a calcium ion channel. Accordingly, anti-CD20 antibodies enhance the host's natural defense mechanism by attacking and killing B cells to which they bind through the CD20 antigen, and such cell death occurs through the complement-dependent cytotoxicity (CDC) pathway and the antibody-dependent cell-mediated cytotoxicity (ADCC) pathway.

To date, representative commercially available anti-CD20 antibodies include rituximab, which is a Type I CD20 antibody, and obinutuzumab, which is a Type II CD20 antibody. Type I antibodies, such as rituximab, translocate CD20 to the Triton-insoluble membrane fraction of the lipid bilayer, whereas Type II antibodies, such as obinutuzumab, induce homotypic aggregation of CD20 at cell-cell contact sites. Furthermore, whereas rituximab does not induce direct cell death (DCD), obinutuzumab efficiently induces DCD through specific binding to CD20. Accordingly, the present inventors prepared an anti-CD20-APEX2 fusion antibody capable of proximity labeling and used the same to search for target proteins involved in DCD, thereby attempting to identify a novel pharmacologically active component capable of exerting a synergistic tumor cell death effect when co-administered with existing CD20 antibodies or administered in a form fused thereto.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop an efficient recombinant antibody, as a therapeutic antibody for B-cell lymphoma, which maintains specific affinity for B-lymphocyte antigens including CD20 while further improving direct cell death activity. As a result, the present inventors first elucidated that MPZL1 (Myelin Protein Zero Like 1) protein is involved in the mechanism of antibody-mediated direct cell death of B-cell lymphoma, and discovered that when concanavalin A, which is a ligand of MPZL1, is conjugated to a B-cell lymphoma-specific antibody, the resulting conjugate can be used as an effective anticancer composition having significantly increased cell death activity.

Accordingly, the objective of the present invention is to provide a recombinant polypeptide that specifically binds to a B lymphocyte and a composition for preventing or treating B-cell lymphoma, comprising the recombinant polypeptide as an active ingredient.

Other objects and advantages of the present invention will become more apparent from the following detailed description, the appended claims, and the accompanying drawings.

### Technical Solution

In one aspect of the present invention, there is provided a recombinant polypeptide that specifically binds to B cells, comprising:
(a) an antibody or an antigen-binding fragment thereof that specifically binds to a B-lymphocyte antigen; and
(b) a concanavalin A protein or a functional fragment thereof bound to the antibody or antigen-binding fragment thereof of (a).

The present inventors have made intensive studies to develop an efficient recombinant antibody, as a therapeutic antibody for B-cell lymphoma, which maintains specific affinity for B-lymphocyte antigens including CD20 and has a further improved direct cell death effect. As a result, the present inventors have first identified that MPZL1 (Myelin Protein Zero Like 1) protein is involved in the mechanism of antibody-mediated direct cell death of B-cell lymphoma, and discovered that when concanavalin A, which is a ligand of MPZL1, is conjugated to a B-cell lymphoma-specific antibody, the resulting conjugate can be used as an effective anticancer composition having significantly increased cell death activity.

As used herein, the term "antibody" refers to a peptide that recognizes and specifically binds to a specific epitope of a B-lymphocyte antigen, for example, CD20, and is intended to encompass not only a complete antibody form but also an antigen-binding fragment (antibody fragment) of a full-length antibody molecule. A complete antibody has a structure with two full-length light chains and two full-length heavy chains, which are linked to each other by a disulfide bond. The heavy chain constant region has the gamma (γ), mu (µ), alpha (α), delta (δ), and epsilon (ε) types and is subclassified into gamma1 (γ1), gamma2 (γ2), gamma3 (γ3), gamma4 (γ4), alpha1 (α1) and alpha2 (α2). The light chain constant region has kappa (κ) and lambda (λ) types.

As used herein, the term "antigen-binding fragment of the antibody" refers to a portion of a polypeptide that has a significant antigen-antibody binding function within a full-length antibody molecule, and includes Fab, F(ab'), F(ab')2, Fv, nanobody or sybody.

Among antibody fragments, Fab has a structure comprising variable regions of a light chain and a heavy chain, a constant region of the light chain, and a first constant region (CH1) of the heavy chain, and has one antigen-binding site.

Fab' differs from Fab in that it has a hinge region containing one or more cysteine residues at the C-terminus of the heavy chain CH1 domain. F(ab')₂ antibodies are generated by formation of disulfide bonds between cysteine residues in the hinge regions of Fab' fragments. Fv is the smallest antibody fragment having only a heavy chain variable region and a light chain variable region. A two-chain Fv has a heavy chain variable region and a light chain variable region linked by a non-covalent bond, whereas a single-chain Fv generally has a heavy chain variable region and a light chain variable region covalently linked through a peptide linker or directly linked at the C-terminus, and thus may form a dimer-like structure similar to a two-chain Fv.

As used herein, the term "heavy chain" refers to both full-length heavy chain and fragments thereof comprising a variable region VH and three constant regions CH1, CH2 and CH3 with the amino acid sequence having sufficient variable region sequence to confer specificity to an antigen.

As used herein, the term "light chain" refers to both full-length light chain and fragments thereof comprising a variable region VL and a constant region CL with the amino acid sequence having sufficient variable region sequence to confer specificity to an antigen.

The variable regions of the heavy and light chains each contain three CDR regions.

As used herein, the term "complementarity determining region (CDR)" refers to the amino acid sequence of the hypervariable region of the heavy and light chains of an immunoglobulin. The heavy chain (HCDR1, HCDR2, and HCDR3) and light chain (LCDR1, LCDR2, and LCDR3) each contain three CDRs, which provide the primary contact residues for an antibody to bind to an antigen or epitope.

The scope of the antibody or antigen-binding fragment of the present invention includes variants having conservative amino acid substitutions in the CDR region. Furthermore, the antibodies or antigen-binding fragments of the present invention may include variants of the amino acid sequence within a range capable of specifically recognizing a particular B-lymphocyte antigen, such as CD20. For example, additional changes can be made to the amino acid sequence of the antibody to further improve the binding affinity and/or other biological properties of the antibody. Such modifications include, for example, deletions, insertions, and/or substitutions of amino acid sequence residues of the antibody. These amino acid modifications are made based on the relative similarity of the amino acid side chain substituents, e.g., hydrophobicity, hydrophilicity, charge, size, etc. By analyzing the size, shape, and type of amino acid side chain substituents, it can be seen that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine are biologically functional equivalents.

In addition, amino acid substitutions in proteins that do not alter the activity of the molecule as a whole are known in the art (H. Neurath, R.L. Hill, The Proteins, Academic Press, New York, 1979). The most common exchanges are between the amino acid residues Ala/Ser, Val/Ile, Asp/Glu, Thr/Ser, Ala/Gly, Ala/Thr, Ser/Asn, Ala/Val, Ser/Gly, Thr/Phe, Ala/Pro, Lys/Arg, Asp/Asn, Leu/Ile, Leu/Val, Ala/Glu, and Asp/Gly.

Considering biologically equivalent variations, the amino acid sequence constituting the antibody of the present invention may encompass sequences having substantial identity to them. Sequences having the substantial identity show at least 61%, specifically at least 70%, more specifically at least 80%, even more specifically at least 90% similarity to the amino acid of this invention, as measured using one of the sequence comparison algorithms. Methods of alignment of sequences for comparison are well-known in the art. Various methods and algorithms for alignment are disclosed in Huang et al., Comp. Appl. BioSci. 8:155-65(1992) and Pearson et al., Meth. Mol. Biol. 24:307-31(1994).

According to a specific embodiment of the present invention, the B-lymphocyte specific antigen is one or more antigens selected from the group consisting of CD19, CD20, CD30 and CD79b.

As used herein, the term "Concanavalin A (ConA)" refers to a carbohydrate-binding lectin protein extracted from jack bean, which specifically binds α-D-mannose and α-D-glucose residues at the non-reducing termini of various sugars, glycoproteins and glycolipids. Like most lectins, ConA is a homotetramer composed of four identical subunits, each of which consists of 235 amino acid residues and has a molecular weight of 26.5 kDa. Each subunit binds to metal ions such as Mn²⁺ or Ca²⁺ and possesses a binding site, and therefore the entire ConA molecule contains a total of four symmetrical binding sites.

As used herein, the term "functional fragment" refers to a fragment of a full-length protein in which some amino acid residues or domains have been deleted, which retains the intrinsic biological activity and function of the full-length protein. For example, a functional fragment of the concanavalin A protein may be a fragment containing one or two of the subunits among the subunits constituting the full-length concanavalin A tetramer, more specifically, it may be a dimer containing two subunits.

According to a specific embodiment of the present invention, the concanavalin A protein or a functional fragment thereof binds to the C-terminus of the antibody or antigen-binding fragment thereof.

According to a specific embodiment of the present invention, the concanavalin A protein or a functional fragment thereof is linked to the C-terminus of the antibody or antigen-binding fragment thereof via a linker.

As used herein, the term "linker" refers to a moiety that physically connects two different fusion partners (e.g., biological polymers) via covalent bonds. The linker may be a peptide linker or a non-peptide linker. When the linker is a non-peptide linker, the linker may be a multi-functional ligand compound having two or more reactive functional groups and capable of binding to two or more molecules, thereby serving as a linker between the molecules. When the linker is a peptide linker, the linker may be composed of three or more amino acids and may form peptide bonds with the respective fusion partners, thereby converting the fusion partners into a single molecule while spacing them apart from each other at an appropriate spatial distance.

More specifically, the linker is a flexible peptide linker represented by (GGGGS)n (where n is an integer from 1 to 5). More specifically, n is an integer from 2 to 5; more specifically, n is an integer from 3 to 5; and most specifically, n is 4.

According to a specific embodiment of the present invention, the functional fragment of the concanavalin A protein is a dimer comprising two monomer subunits that constitute a concanavalin A tetramer.

According to a specific embodiment of the present invention, the two monomer subunits of concanavalin A bind to the C-terminus of each heavy chain of the antibody that specifically binds to the B-lymphocyte antigen.

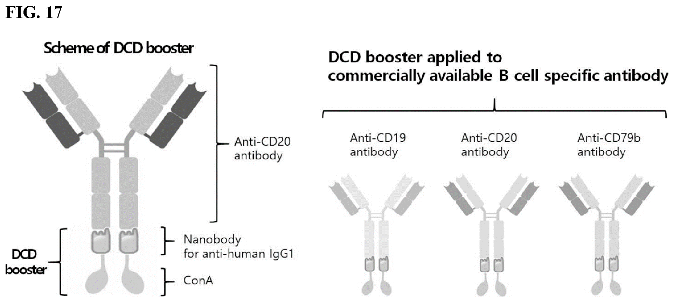

When a functional fragment of concanavalin A, namely a homodimer, is bound to a B-lymphocyte antigen-specific antibody, as shown in FIG. 17, each of the two monomers may be bound to the C-terminus of a heavy chain constant region of the antibody.

According to a specific embodiment of the present invention, the monomer of concanavalin A of the present invention comprises the amino acid sequence of SEQ ID NO: 17.

According to a specific embodiment of the present invention, the B-lymphocyte specific antigen is CD20.

More specifically, the antibody that specifically binds to CD20 may be obinutuzumab or rituximab. In this case, the "antigen-binding fragment of an antibody that specifically binds to a B-lymphocyte antigen" may be a fragment of a full-length antibody comprising all or some of the six CDRs (HCDR1 to HCDR3 and LCDR1 to LCDR3) of obinutuzumab or rituximab. The amino acid sequences of rituximab and obinutuzumab may be obtained from various public databases, and may be obtained, for example, from U.S. Patent Application Publication No. 2015-0141620 for rituximab and U.S. Patent No. 11,110,087 for obinutuzumab, respectively.

When the antibody of the present invention that specifically binds to CD20 is obinutuzumab, the antibody or antigen-binding fragment thereof may comprise a heavy chain variable region comprising the HCDR1 region of SEQ ID NO: 1, the HCDR2 region of SEQ ID NO: 2 and the HCDR3 region of SEQ ID NO: 3.

According to a specific embodiment of the present invention, the antibody or its antigen-binding fragment may further comprise a light chain variable region comprising the LCDR1 region of SEQ ID NO: 4; the LCDR2 region of SEQ ID NO: 5; and the LCDR3 region of SEQ ID NO: 6.

More specifically, the antibody or antigen-binding fragment may include the heavy chain variable region of SEQ ID NO: 7 and/or the light chain variable region of SEQ ID NO: 8.

When the antibody of the present invention that specifically binds to CD20 is rituximab, the antibody or antigen-binding fragment thereof may comprise a heavy chain variable region comprising the HCDR1 region of SEQ ID NO: 9, the HCDR2 region of SEQ ID NO: 10, and the HCDR3 region of SEQ ID NO: 11.

According to a specific embodiment of the present invention, the antibody or its antigen-binding fragment may further comprise a light chain variable region comprising the LCDR1 region of SEQ ID NO: 12; the LCDR2 region of SEQ ID NO: 13; and the LCDR3 region of SEQ ID NO: 14.

More specifically, the antibody or antigen-binding fragment may include the heavy chain variable region of SEQ ID NO: 15 and/or the light chain variable region of SEQ ID NO: 16.

According to a specific embodiment of the present invention, the recombinant polypeptide of the present invention may further comprise an IgG1-specific nanobody. In this case, one or two nanobodies may be included, and they may be bound to the C-terminus of the concanavalin A protein that is bound to the B-lymphocyte antigen-specific antibody, or they may be positioned between the B-lymphocyte antigen-specific antibody and the concanavalin A protein.

In another aspect of the present invention, there is provided a nucleic acid molecule encoding the recombinant polypeptide of the present invention.

As used herein, the term "nucleic acid molecule" is meant to encompass DNA (gDNA and cDNA) and RNA molecules. Nucleotides, which are the basic structural units in nucleic acid molecules, include not only natural nucleotides, but also analogues having modified sugar or base moieties (Uhlman and Peyman, Chemical Reviews, 90:543-584 (1990)). It will be clear to those skilled in the art that the nucleotide sequence encoding the recombinant polypeptide of the present invention may involve modifications including additions, deletions, or non-conservative or conservative substitutions of nucleotides.

The nucleic acid molecule of the present invention is interpreted to include a nucleotide sequence that is substantially identical to any of the above nucleotide sequences. Substantial identity means a sequence showing at least 80% homology, specifically at least 90% homology, more specifically at least 95% homology as determined by aligning the sequence of the present invention with any other sequence to correspond to each other as much as possible and analyzing the aligned sequence using an algorithm commonly used in the art.

According to a specific embodiment of the present invention, the nucleic acid molecule of the present invention may be an mRNA molecule, and more specifically, it may be an IVT (in vitro transcribed) mRNA.

When mRNA is used as the nucleic acid molecule of the present invention, various modifications may be applied to improve the expression (translation) efficiency of the recombinant polypeptide of the present invention; for example, altering the length of the poly(A) tail or substituting some of the adenine bases; modifying the 5' cap; or incorporating one or more modified nucleosides. Examples of modified nucleosides that can be applied include, for example, N1-methylpseudouridine, pseudouridine, 2-thiouridine, 5-methyluridine, 5-methylcytidine and 5-methoxyuridine, but are not limited thereto; any modified nucleoside known in the art to reduce the immunogenicity of mRNA molecules may be used.

In another aspect of the present invention, there is provided a gene delivery system comprising the nucleic acid molecule of the present invention.

According to the present invention, the recombinant polypeptide of the present invention may be recombinantly obtained *in vitro* by expressing a nucleic acid molecule encoding the same in a host cell, or the nucleic acid molecule encoding the recombinant polypeptide may be delivered into the body of a patient as an active ingredient, thereby allowing the recombinant polypeptide of the present invention to be produced through the patient's expression system.

As used herein, the term "express" refers to being artificially replicated as an extrachromosomal factor or by chromosomal integration in a target cell via a gene delivery system to cause the target cell to express an exogenous gene or overexpress an endogenous gene. Accordingly, "express" may be used interchangeably with "transformation", "transfection", or "transduction". More specifically, "to express" in the present invention refers to cause a target cell to artificially express an exogenous gene.

The term "gene delivery system" or "gene delivery vehicle" as used herein refers to any means for delivering a gene into a cell, and the term "gene delivery" has the same meaning as intracellular transduction of the gene. At the cell or tissue level, gene delivery has the same meaning as spread of the gene. Thus, the gene delivery system of the present invention may be referred to as a gene transduction system or a gene spread system.

To prepare a gene delivery vector of the present invention, the nucleotide sequence of the present invention is present in an appropriate expression construct, and, within the expression construct, the nucleotide sequence of the present invention may be operatively linked to an expression regulatory sequence. As used herein, the term "operatively linked" refers to a functional linkage between a nucleic acid expression regulatory sequence (e.g., a promoter, a signal sequence, or an array of transcription regulation factor binding sites) and the target nucleic acid sequence. Through the linkage, the regulatory sequence regulates the transcription and/or translation of the target nucleic acid sequence.

The recombinant vector system of the present invention may be constructed by various methods known in the art, and specific methods therefor are disclosed, for example, in Sambrook et al., Molecular Cloning, A Laboratory Manual, Cold Spring Harbor Laboratory Press (2001).

The vector of the present invention may typically be constructed as a cloning vector or an expression vector. In addition, the vector of the present invention may be constructed using a prokaryotic cell or a eukaryotic cell as a host. For example, when the vector of the present invention is an expression vector and a prokaryotic cell is used as a host, the vector generally includes a strong promoter capable of driving transcription (e.g., a tac promoter, lac promoter, lacUV5 promoter, lpp promoter, pLλ promoter, pRλ promoter, rac5 promoter, amp promoter, recA promoter, SP6 promoter, trp promoter and T7 promoter etc.), a ribosome binding site for initiation of translation and transcription/translation termination sequences. When E. coli (e.g., HB101, BL21 and DH5α etc.), is used as the host cell, the promoter and operator region of the E. coli tryptophan biosynthesis pathway (Yanofsky, C. J. Bacteriol. 158:1018-1024 (1984)) and the leftward promoter of phage λ (pLλ promoter, Herskowitz, I. et al., Ann. Rev. Genet. 14:399-445 (1980)) may be used as regulatory regions. When a Bacillus species is used as the host cell, the promoter of a toxin protein gene of Bacillus thuringiensis (Appl. Environ. Microbiol. 64:3932-3938 (1998); Mol. Gen. Genet. 250:734-741 (1996)) or any promoter capable of expression in Bacillus species may be used as a regulatory region.

Meanwhile, the recombinant vectors of the present invention may be produced by manipulating plasmids commonly used in the art (e.g., pCL, pSC101, pGV1106, pACYC177, ColE1, pKT230, pME290, pBR322, pUC8/9, pUC6, pBD9, pHC79, pIJ61, pLAFR1, pHV14, the pGEX series, the pET series, and pUC19, etc.), phages (e.g., λgt4·λB, λ-Charon, λΔz1, and M13, etc.) or viruses (e.g., SV40, etc.).

On the other hand, when the expression vector of the present invention uses a eukaryotic cell as a host, a promoter derived from the genome of a mammalian cell (i.e., a metallothionein promoter, β-actin promoter, human hemoglobin promoter, or human muscle creatine promoter, or a promoter derived from a mammalian virus, such as an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter or Rous sarcoma virus (RSV) promoter) may be used.

The recombinant vector of the present invention may further comprise another sequence fused thereto in order to facilitate purification of the antibody expressed therefrom. Examples of the sequence to be fused include glutathione S-transferase (Pharmacia, USA), maltose-binding protein (NEB, USA), FLAG (IBI, USA), 6× His (hexahistidine; Quiagen, USA), tag sequences such as Pre-S1 and c-Myc and leader sequences such as OmpA and PelB. Furthermore, since the protein expressed by the vector of the present invention is an antibody, the expressed antibody can be easily purified using a protein A column or the like, even without an additional sequence for purification.

Meanwhile, the recombinant vector of the present invention may include antibiotic resistance genes commonly used in the art as a selection marker; for example, genes conferring resistance to ampicillin, gentamicin, carbenicillin, chloramphenicol, streptomycin, kanamycin, geneticin, neomycin and tetracycline.

A vector expressing the recombinant polypeptide of the present invention may be either a vector system in which the light chain and the heavy chain of the antibody included in the recombinant polypeptide are simultaneously expressed from a single vector or a system in which the light chain and the heavy chain are expressed from separate vectors, respectively. In the latter case, the two vectors are introduced into a host cell through co-transformation and targeted transformation. Co-transformation is a method in which respective vector DNAs encoding the light chain and the heavy chain are simultaneously introduced into a host cell, followed by selecting cells expressing both the light chain and the heavy chain. Targeted transformation is a method in which cells transformed with a vector comprising the light chain (or heavy chain) are selected, and the selected cells expressing the light chain are transformed again with a vector comprising the heavy chain (or light chain), thereby finally selecting cells expressing both the light chain and the heavy chain.

The gene delivery vector of the present invention may be any gene delivery system commonly used for gene insertion, and examples thereof include, but are not limited to, plasmids, adenoviruses, adeno-associated viruses (AAV), retroviruses, lentiviruses, herpes simplex viruses, vaccinia viruses, liposomes, niosomes and lipid nanoparticles.

In still another aspect of the present invention, there is provided a host cell transformed with the gene delivery system of the present invention.

Host cells capable of stably and continuously cloning and expressing the gene delivery system of the present invention are known in the art, and any host cell known in the art may be used. For example, in the case of prokaryotic cells, examples thereof include, but are not limited to, *Escherichia coli*; strains of the genus *Bacillus* such as *Bacillus subtilis* and *Bacillus thuringiensis*; *Streptomyces*; *Pseudomonas*, for example, *Pseudomonas putida*; *Proteus mirabilis*; or *Staphylococcus*, for example, *Staphylococcus carnosus*.

Suitable eukaryotic host cells that may be used in the present invention may include, for example, fungi such as *Aspergillus species*; yeasts such as *Pichia pastoris*, *Saccharomyces cerevisiae* and *Schizosaccharomyces*; insect-derived cells; plant-derived cells; or mammalian-derived cells such as CHO (Chinese hamster ovary) cells.

The cultivation of host cells for producing an antibody or antigen-binding fragment thereof may be carried out according to suitable media and culture conditions known in the art. Such a cultivation process can be readily adjusted and used by a person skilled in the art depending on the selected strain, and specific examples of cultivation methods are disclosed in various references, for example, James M. Lee, Biochemical Engineering, Prentice-Hall International Editions, pp. 138-176. The antibody obtained by culturing the host cells may be used without purification, or may be further purified to high purity using various conventional methods, such as dialysis, salt precipitation and chromatography. When chromatography is used, the type and order of columns may be selected from columns for ion-exchange chromatography, size-exclusion chromatography and affinity chromatography, depending on the characteristics of the antibody and the method of culturing the host cells.

In still another aspect of the invention, there is provided a composition for preventing or treating a B-cell lymphoma, comprising the recombinant polypeptide, the nucleic acid molecule or the gene delivery system of the present invention as an active ingredient.

In still another aspect of the invention, there is provided a method for preventing or treating a B-cell lymphoma, comprising administering to a subject in need thereof the recombinant polypeptide, the nucleic acid molecule or the gene delivery system of the present invention as an active ingredient.

As used herein, the term "preventing" refers to inhibiting the occurrence of a disorder or disease in a subject who has never been diagnosed as having the disorder or disease, but is at risk of developing the condition or disease.

As used herein, the term "treating" refers to (a) inhibiting the progress of a disorder, disease or symptom; (b) alleviating the disorder, disease or symptom; or (c) eliminating the disorder, disease or symptom. The composition of the present invention, when administered to a subject, significantly blocks proliferation of B-cell lymphoma specifically expressing a B-lymphocyte antigen including CD20 on the surface thereof and strongly induces direct cell death, thereby inhibiting the progression of symptoms caused by B-cell lymphoma, or eliminating or alleviating such symptoms. Therefore, the composition of the present invention may itself be a cell therapy composition, or may be applied as a therapeutic adjuvant for the disease by being administered together with other active ingredients. Accordingly, the term "treatment" or "therapeutic agent" in the present specification includes the meaning of "therapeutic aid" or "therapeutic adjuvant".

As used herein, the terms "administration" or "administering" refer to directly administering a therapeutically effective amount of the composition of the present invention to a subject, such that the same amount is formed in the body of the subject.

As used herein, the term "therapeutically effective amount" refers to an amount of the pharmaceutical composition of the present invention in which the pharmacologically active ingredient is contained in an amount sufficient to provide a therapeutic or preventive effect to the subject to whom the composition is to be administered, and thus includes the meaning of a "preventively effective amount."

As used herein, the term "subject" includes without limitation, humans, mice, rats, guinea pigs, dogs, cats, horses, cattle, pigs, monkeys, chimpanzees, baboons, or rhesus monkeys. Specifically, the subject of the present invention is a human.

According to a specific embodiment of the present invention, the B-cell lymphoma that can be prevented or treated with the composition of the present invention is selected from the group consisting of diffuse large B-cell lymphoma (DLBCL), follicular lymphoma, marginal zone B-cell lymphoma (MZL), small lymphocytic lymphoma (SLL), and mantle cell lymphoma (MCL).

In still another aspect of the invention, there is provided a composition for preventing or treating a B-cell lymphoma, comprising:
(a) an antibody or antigen-binding fragment thereof that specifically binds to a B-lymphocyte antigen, or a nucleic acid molecule encoding the same; and
(b) MPZL1 protein or a nucleic acid molecule encoding the same.

In still another aspect of the invention, there is provided a method for preventing or treating B-cell lymphoma, comprising administering to a subject in need thereof the following:
(a) an antibody or antigen-binding fragment thereof that specifically binds to a B-lymphocyte antigen, or a nucleic acid molecule encoding the same; and
(b) MPZL1 protein or a nucleic acid molecule encoding the same.

Since the antibody specifically binding to a B-lymphocyte antigen, the antigen-binding fragment thereof and the nucleic acid molecule encoding the same used in the present invention have already been described above, description thereof will be omitted to avoid excessive overlapping.

According to the present invention, the present inventors have first demonstrated that MPZL1 protein is involved in the mechanism of direct cell death of B-cell lymphoma mediated by an antibody, and confirmed that overexpression of MPZL1 significantly increases death of lymphoma cells. Accordingly, when a B-lymphocyte antigen-specific antibody including an antibody specific to CD20 and MPZL1 protein (or a nucleic acid molecule encoding the same) are administered in combination, a remarkable synergistic antitumor effect can be achieved.

### Advantageous Effects

The features and advantages of the present invention are summarized as follows:
(a) The present invention provides a recombinant polypeptide that specifically binds to B lymphocytes and a composition for preventing or treating B-cell lymphoma comprising the recombinant polypeptide as an active ingredient.
(b) The present invention can significantly enhance direct cytotoxic activity against tumor cells by conjugating concanavalin A, which is a ligand of MPZL1, to an antibody molecule that recognizes a B- lymphocyte antigen such as CD20.
(c) Accordingly, unlike therapeutic antibodies that act mainly through antibody-dependent cell-mediated cytotoxicity (ADCC), complement-dependent cytotoxicity (CDC) and antibody-dependent cellular phagocytosis (ADP), the recombinant polypeptide of the present invention does not rely on the effector mechanisms of a patient's immune system. Therefore, the recombinant polypeptide can be usefully employed as an effective therapeutic composition even for cancer patients with reduced immune activity.

### Brief Description of Drawings

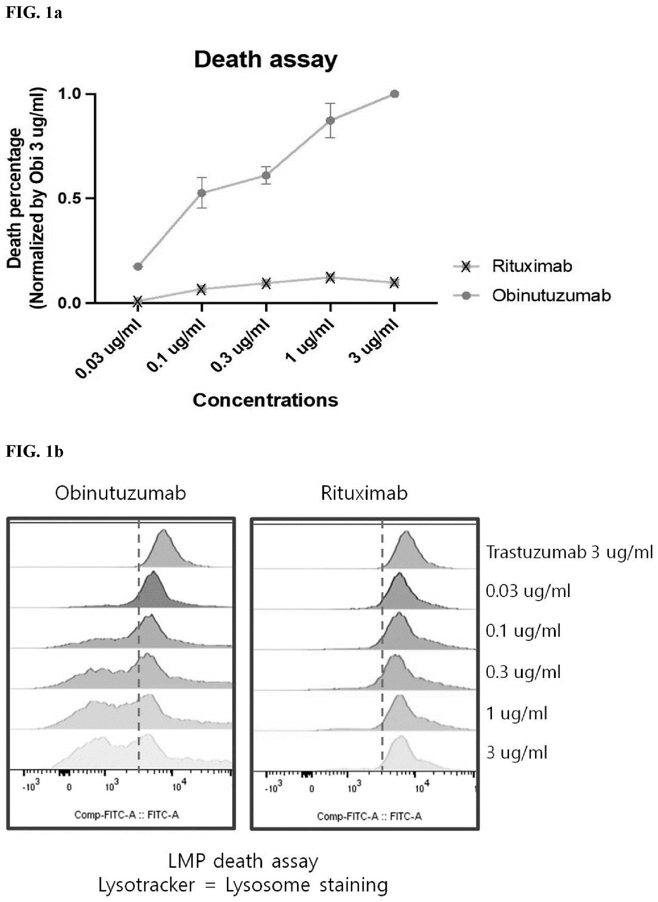

FIG. 1 illustrates the results of a cell death assay using commercially available anti-CD20 antibodies, and shows the results of cell death rate analysis after treatment of B-cell lymphoma cells with various concentrations of rituximab and obinutuzumab (FIG. 1a) and the results of LMP (lysosome membrane permeabilization) measurement by flow cytometry (FIG. 1b).

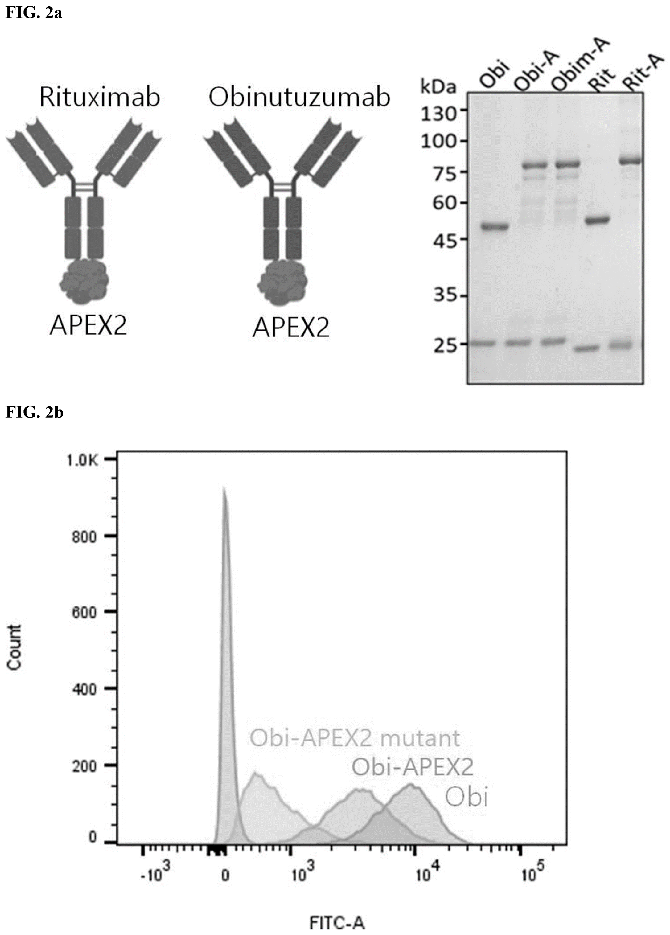

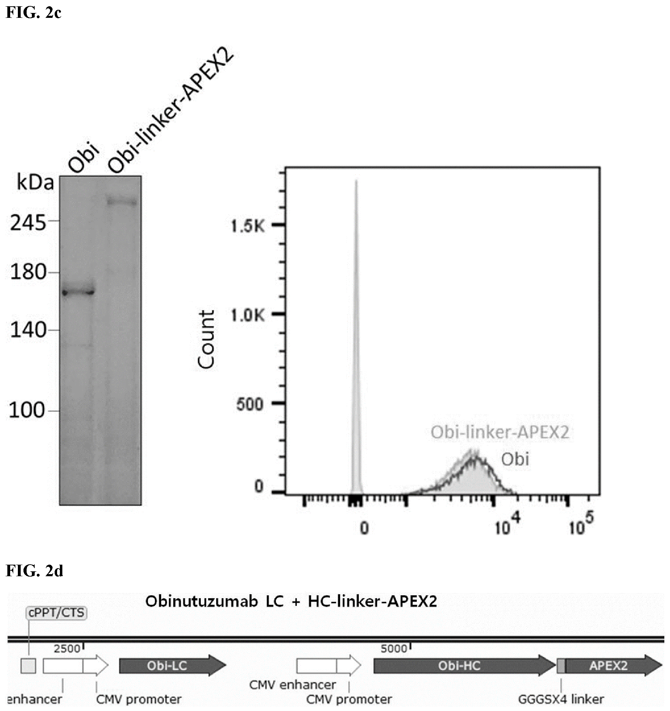

FIG. 2 illustrates the results of preparing an APEX2 fusion antibody for proximity labeling and verifying the function thereof, and respectively shows the binding affinity and binding assay results, obtained by flow cytometry, of an APEX2 fusion antibody and a negative control antibody having an LC-N97A mutation, in a case where APEX2 and an anti-CD20 antibody are fused without a linker (FIG. 2a) and in a case where APEX2 and an anti-CD20 antibody are linked through a linker (FIG. 2c). FIG. 2d is a schematic diagram of an expression construct of a fusion antibody in which APEX2 and an anti-CD20 antibody (obinutuzumab) are linked via a GGGS × 4 linker.

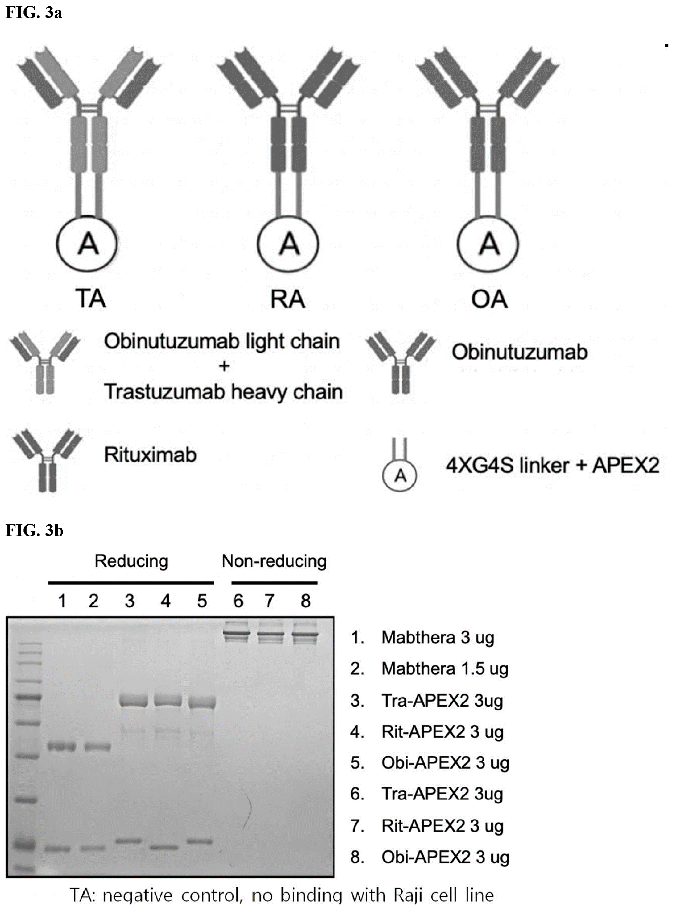

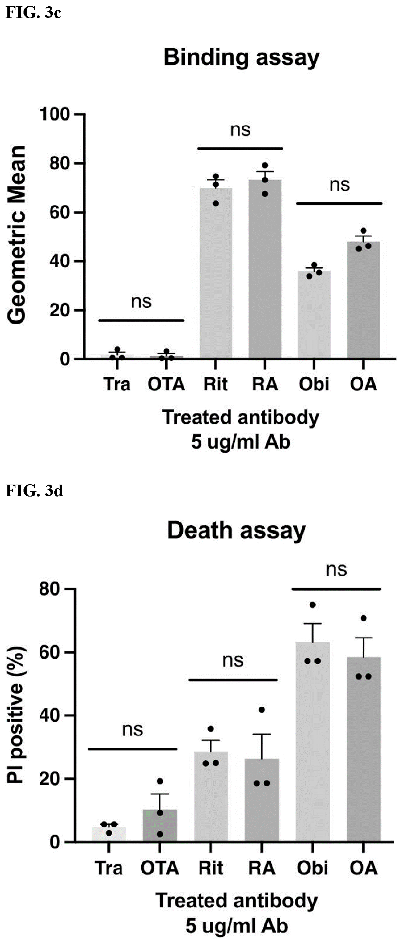

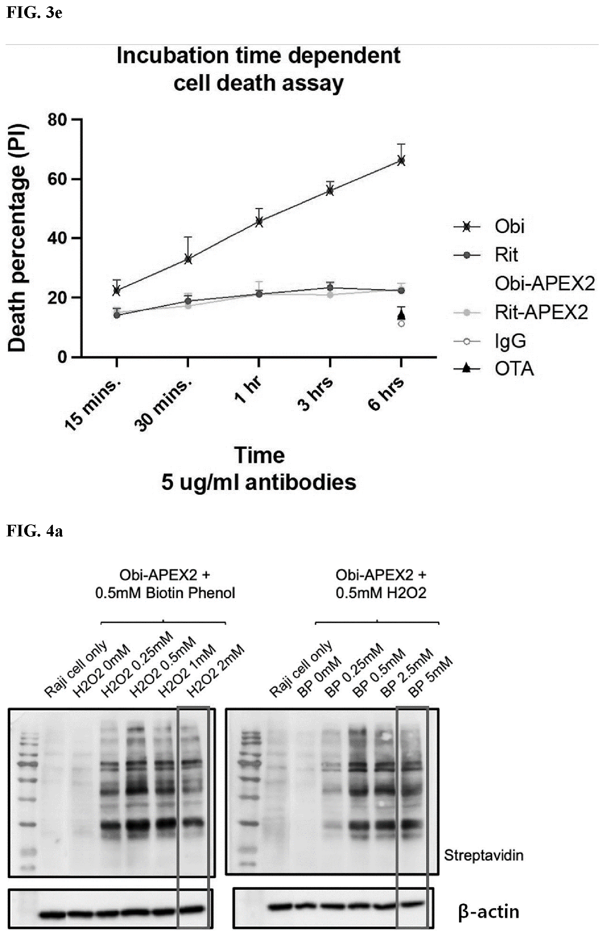

FIG. 3 illustrates the results of preparing a second-generation APEX2 fusion antibody for proximity labeling and verifying the function thereof, and shows a schematic diagram of a fusion antibody in which the heavy chain of obinutuzumab, rituximab or trastuzumab is linked to APEX2 via a flexible GGGGS × 4 linker (FIG. 3a), SDS-PAGE analysis results confirming the expression and purification of the APEX2 fusion antibody under reducing and non-reducing conditions (FIG. 3b), the binding ability of the APEX2 fusion antibody to CD20 (FIG. 3c), cell death-inducing ability (FIG. 3d), and changes in cell death rate over time (FIG. 3e).

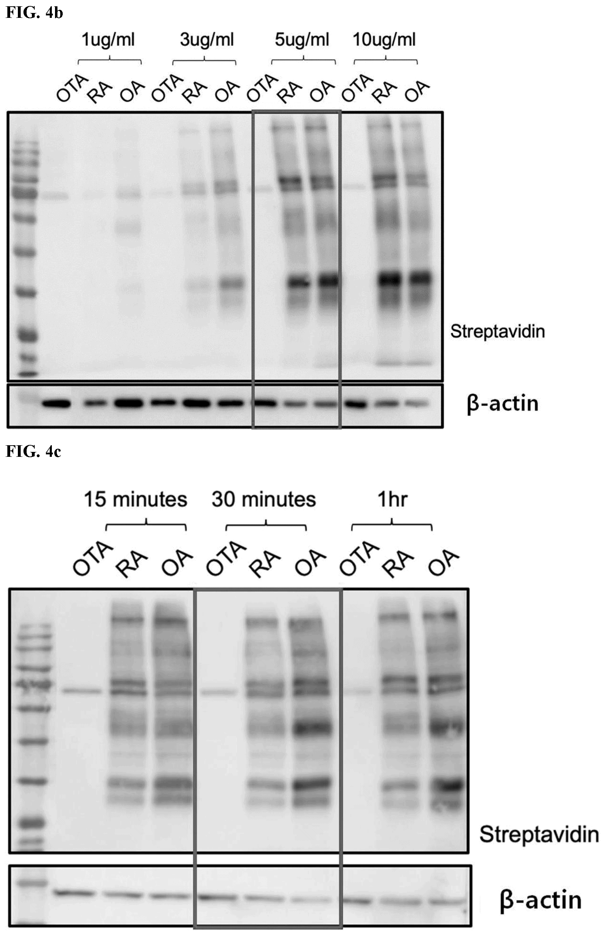

FIG. 4 shows the results of biotinylation optimization using the APEX2 fusion antibody. Protein biotinylation efficiency was confirmed according to the concentrations of biotin phenol and H₂O₂ (FIG. 4a), biotinylation efficiency according to changes in antibody concentration (FIG. 4b) and antibody incubation time (FIG. 4c) was confirmed by applying the optimal concentrations of biotin phenol and H₂O₂ selected based thereon.

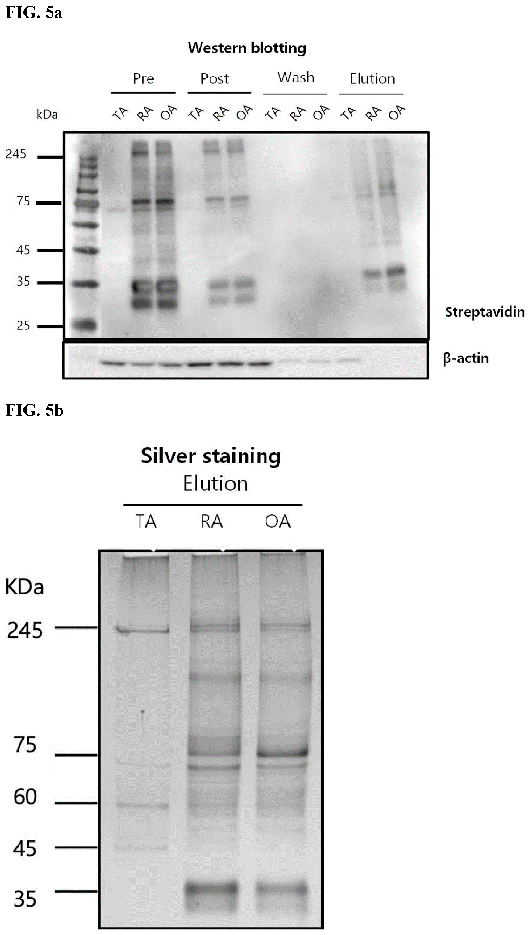

FIG. 5 shows Western blotting results (FIG. 5a) and silver staining results (FIG. 5b), each showing the purification results of biotinylated proteins.

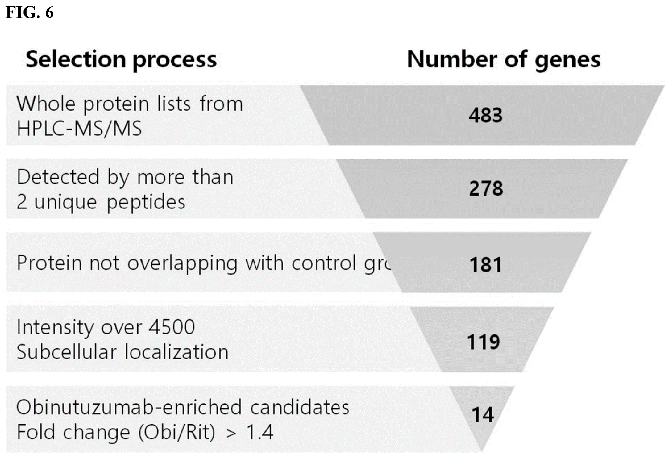

FIG. 6 shows the process of selecting protein candidates detected by HPLC-MS/MS, in which 14 membrane proteins showing an expression change of 1.4-fold or greater were selected as final candidates among the proteins detected using Bruker TIMS-TOF PRO2.

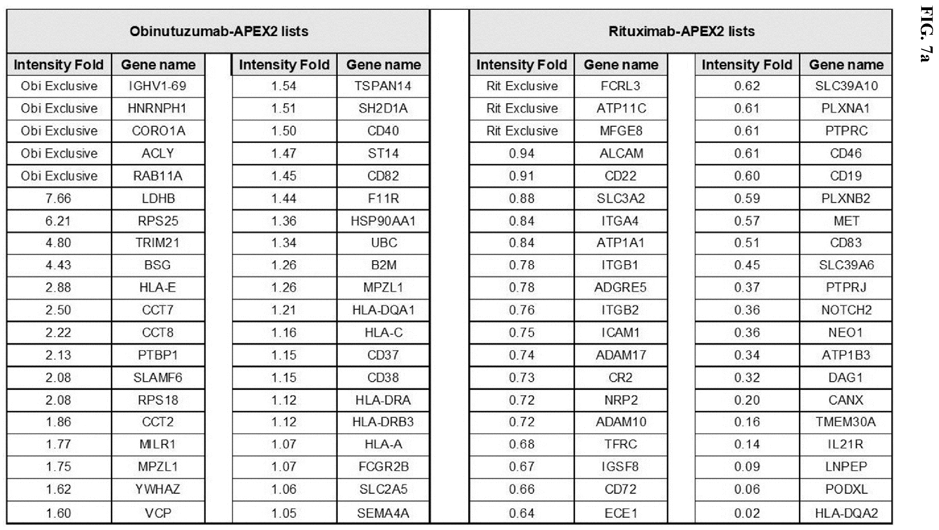

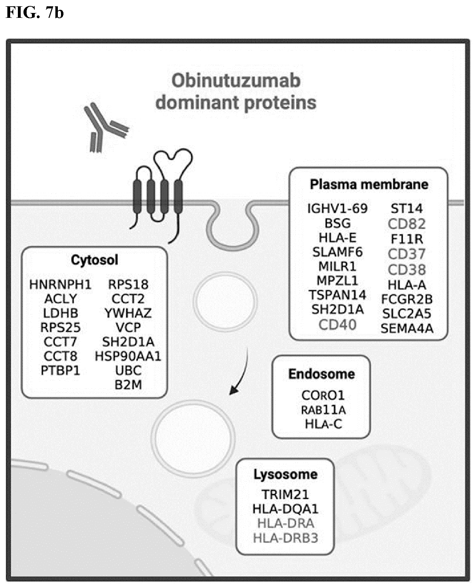

FIG. 7a shows a target list selected through HPLC-MS/MS analysis, in which fold change values obtained by dividing intensity values in protein samples obtained using obinutuzumab-linker-APEX2 by intensity values obtained using rituximab-linker-APEX2 are shown. FIG. 7b shows a schematic diagram of the intracellular localization of proteins showing high intensity fold values in obinutuzumab.

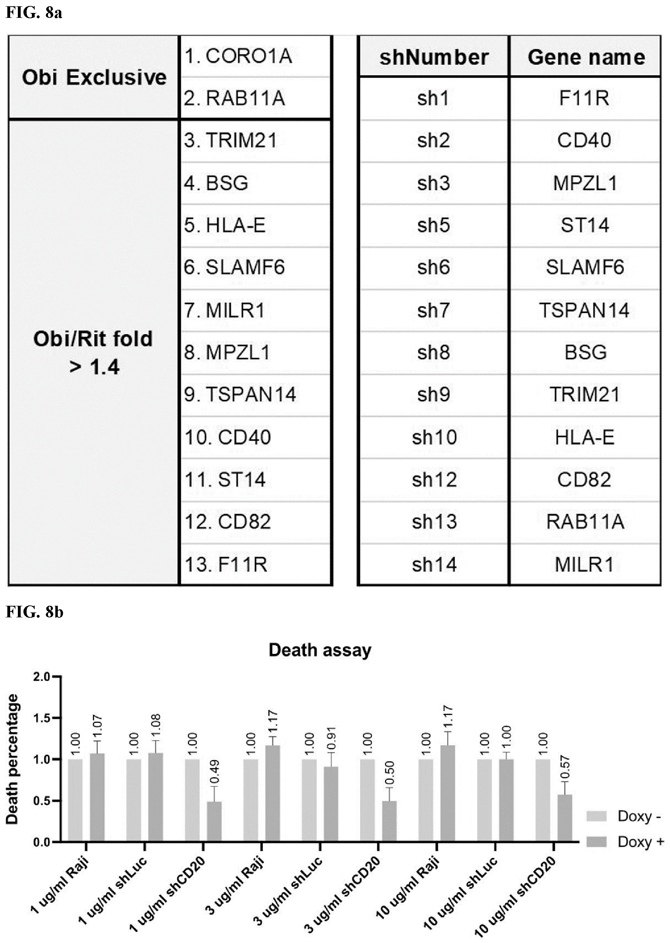

FIG. 8 shows a list of target genes selected to identify proteins involved in direct cell death (DCD) induced by obinutuzumab (FIG. 8a) and the results of an LMP assay confirming the effect on the mechanism of direct cell death induced by obinutuzumab by suppressing protein expression using doxycycline-induced shRNA (FIG. 8b), respectively.

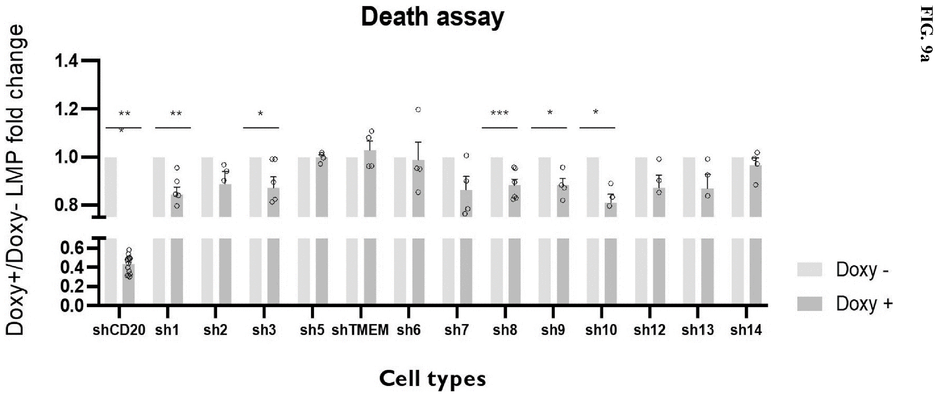

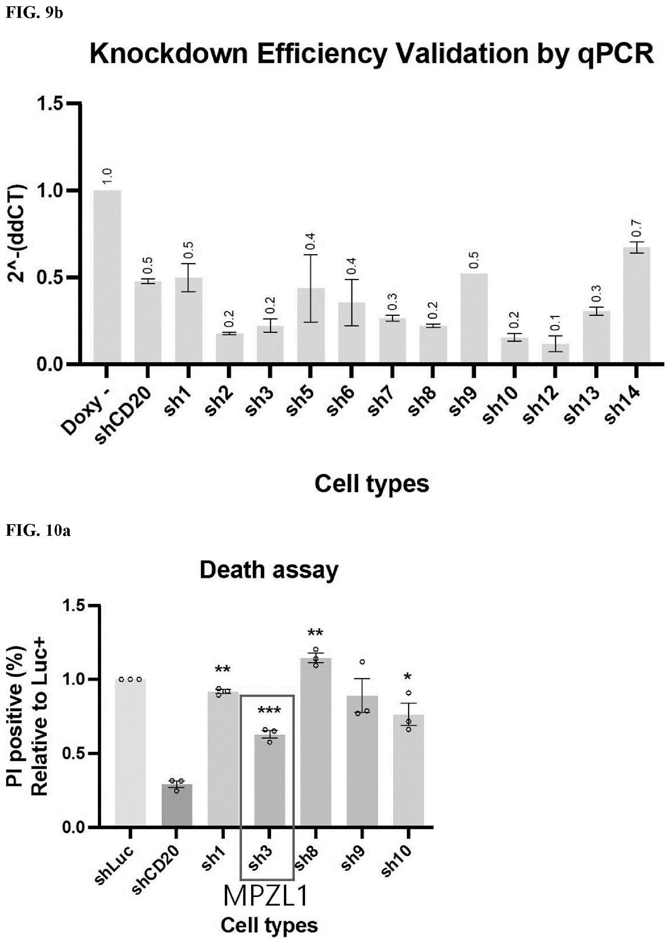

FIG. 9 shows the results of an LMP assay analyzing the viability of Raji cells treated with doxycycline-induced shRNA targeting selected candidate genes (FIG. 9a) and the results of qPCR confirming the knockdown efficiency by the shRNA (FIG. 9b), respectively.

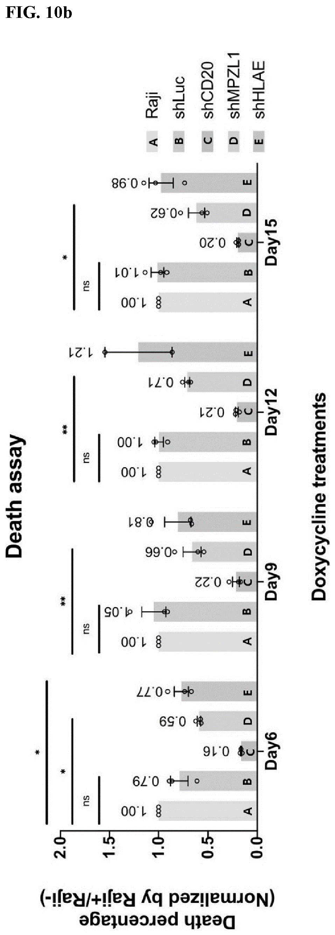

FIG. 10 shows the results of a direct cell death (DCD) assay performed using candidates that showed significant results in the preceding LMP assay (FIG. 10a) and the results of observing cell death according to the duration of doxycycline treatment for MPZL1 and HLA-E, which are the top two genes most affected in terms of cell death compared to the double control group (FIG. 10b), respectively.

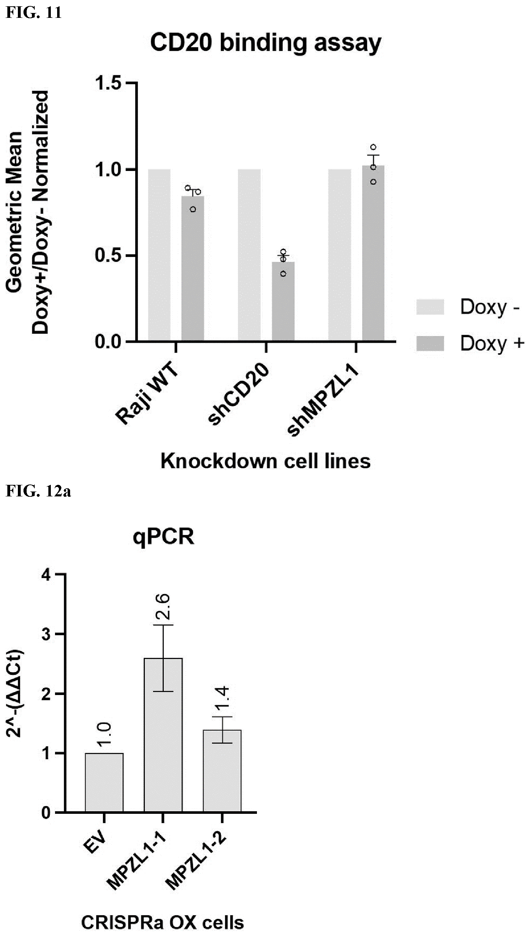

FIG. 11 shows the results of a CD20 binding assay analyzing differences in CD20 expression levels and CD20 binding ability following MPZL1 knockdown.

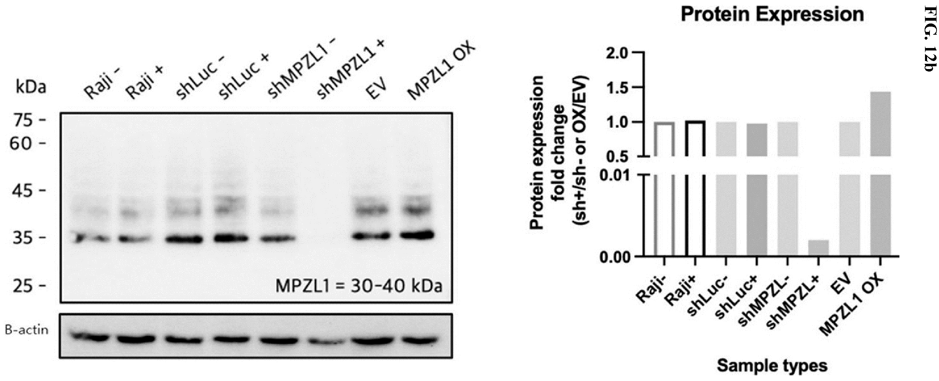

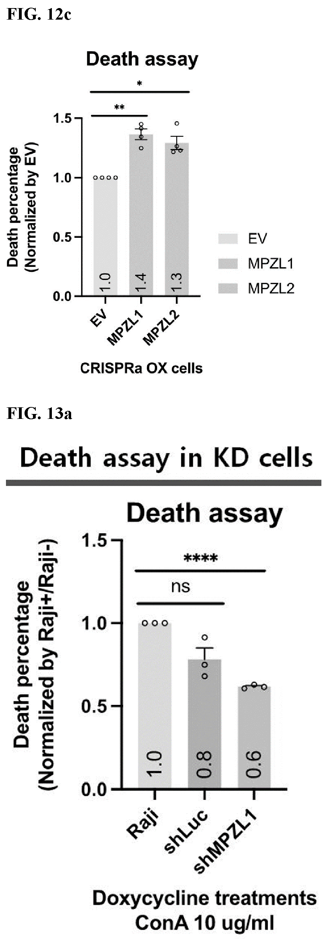

FIG. 12 shows the results of qPCR measurement of MPZL1 RNA expression levels in MPZL1-overexpressing cells (FIG. 12a) and the results of Western blot analysis of protein expression levels in MPZL1-knockdown and MPZL1-overexpressing cells (FIG. 12b), respectively.

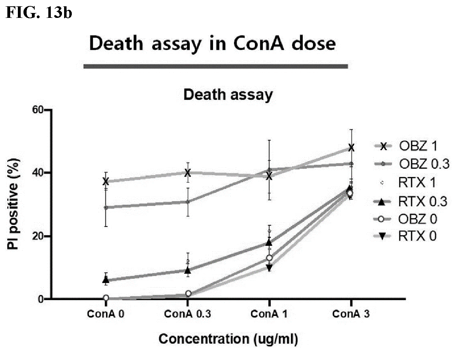

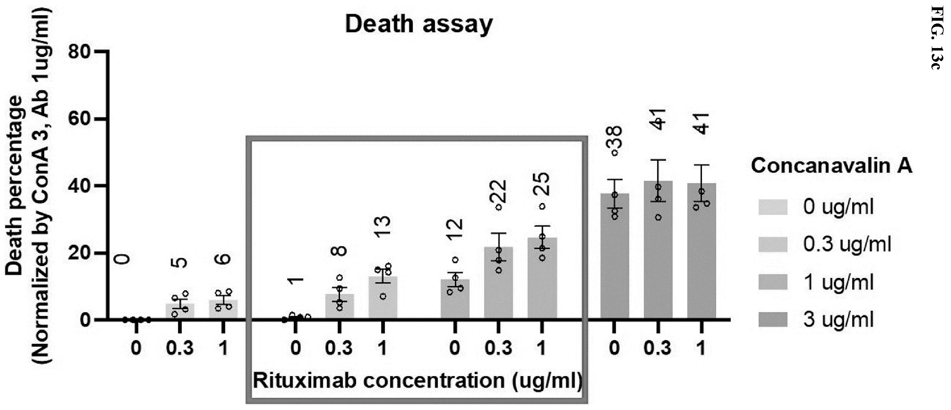

FIG. 13a shows the results of observing changes in cell death rate following treatment of Raji cells with doxycycline-inducible shRNA targeting MPZL1, which is a ligand of concanavalin A. FIGS. 13b and 13c show the results of comparing the direct cell death (DCD)-inducing efficiency of anti-CD20 antibodies according to the concentration of concanavalin A.

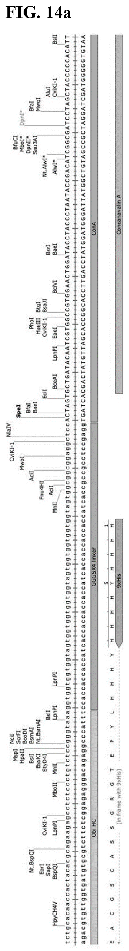

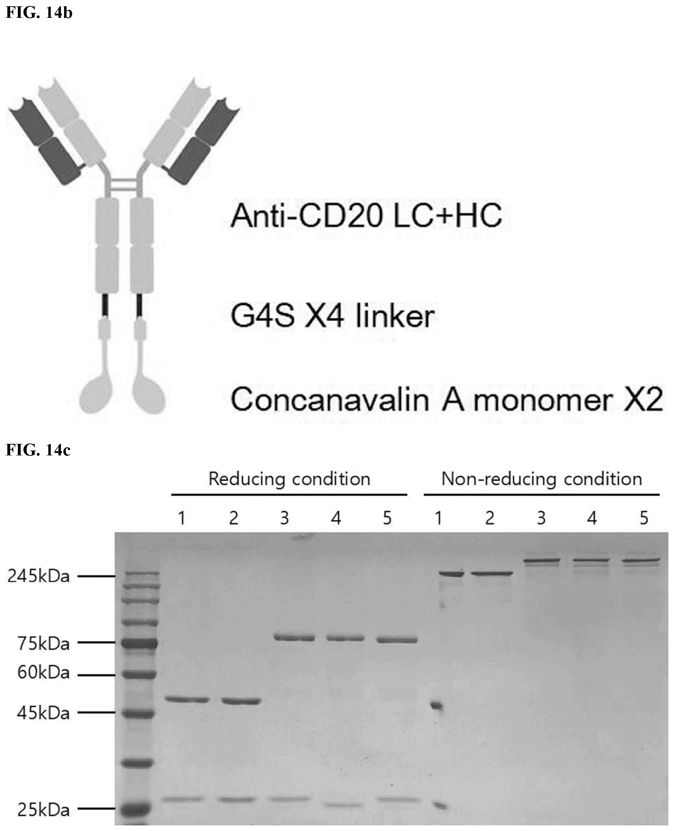

FIG. 14 shows the expression construct (FIG. 14a), schematic diagram (FIG. 14b), and SDS-PAGE analysis results of the purified fusion antibody (FIG. 14c), respectively, for the concanavalin A-fused anti-CD20 antibody.

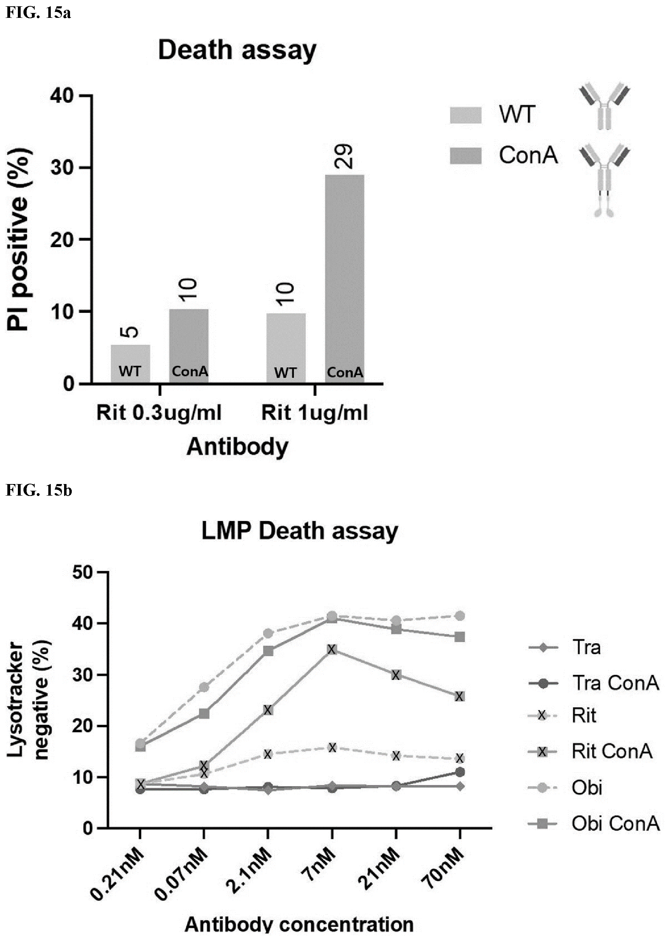

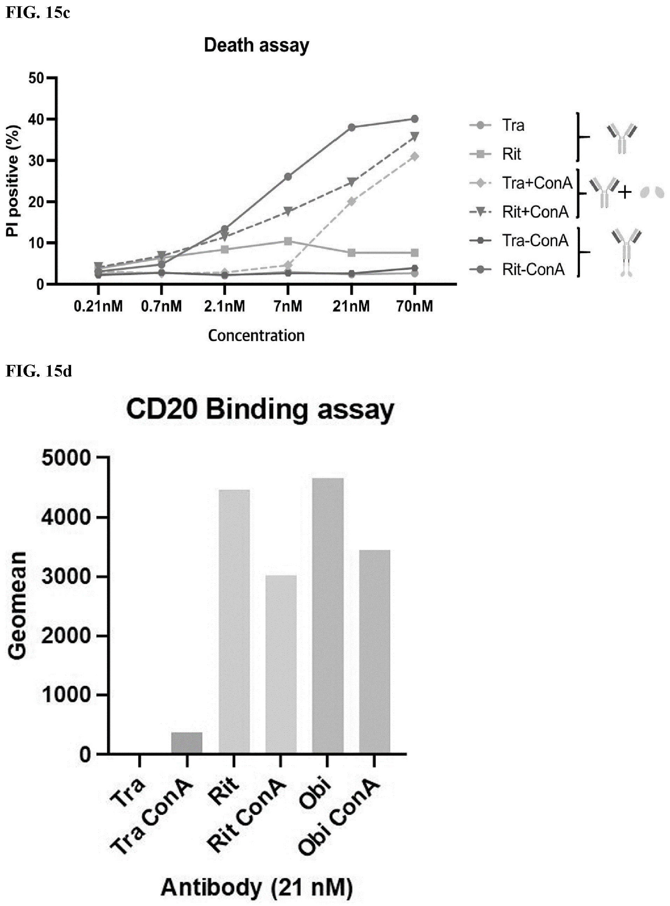

FIG. 15a shows the results of comparing the cell death-inducing effects of rituximab and rituximab-concanavalin A (Rit-ConA). FIG. 15b shows the results of comparing the cell death-inducing effects of three wild-type antibodies and fusion antibodies thereof. FIG. 15c shows the results of comparing the effects and toxicity of combined administration of concanavalin A and an anti-CD20 antibody with those of a fusion antibody in which concanavalin A and the anti-CD20 antibody are fused into a single molecule. FIG. 15d shows the results of comparing the antigen-binding ability of three wild-type antibodies and fusion antibodies thereof.

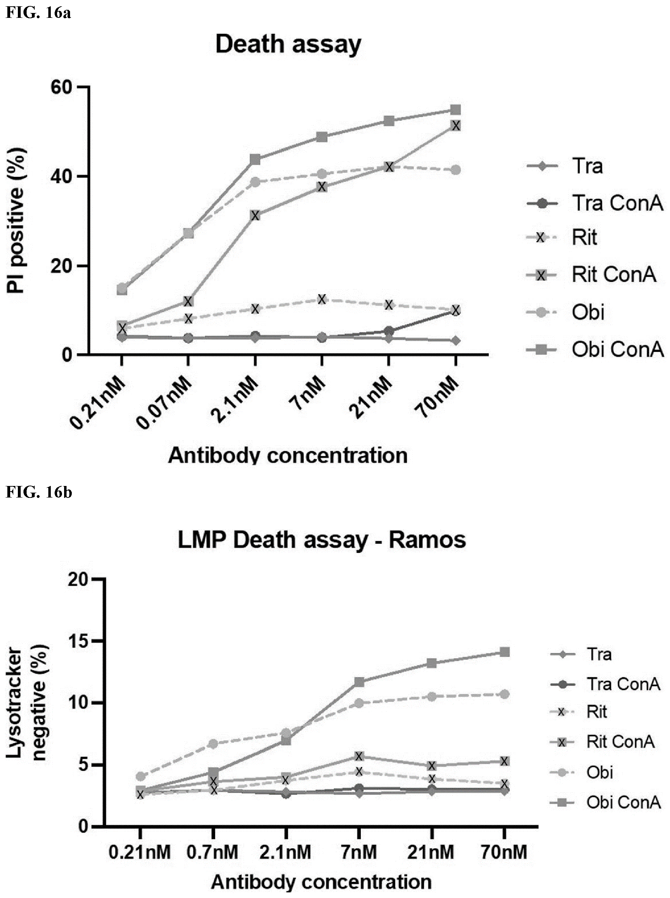

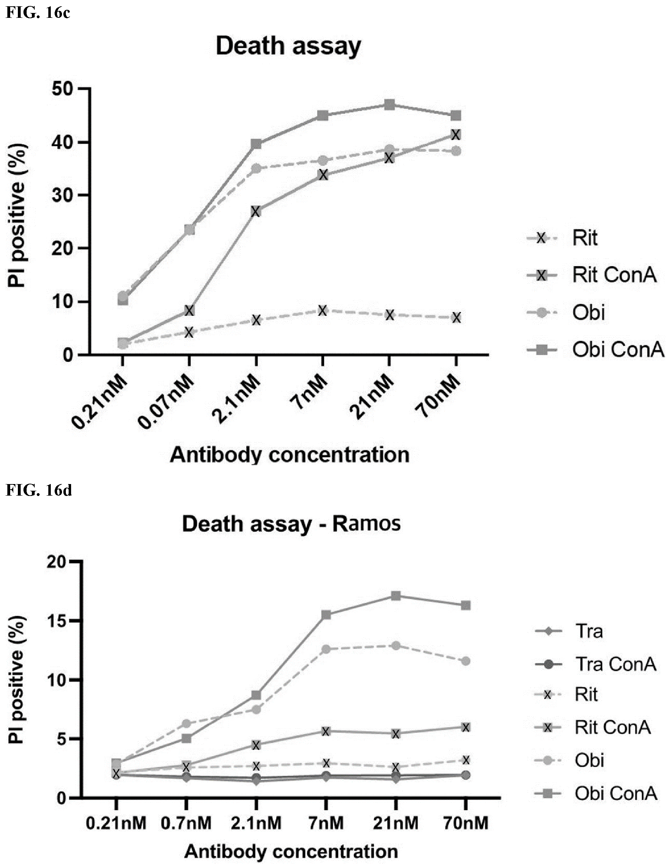

FIG. 16 shows the results of investigating the cell death-inducing effects of concanavalin A-conjugated antibodies on Raji cells (FIGS. 16a and 16b) and Ramos cells (FIGS. 16c and 16d), respectively.
FIG. 17 shows a schematic diagram of various B-cell specific recombinant antibodies of the present invention conjugated with concanavalin A.

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Example

### Experimental Methods

### Cells

CHO-K1 and HEK293T cells were purchased from the Korean Cell Line Bank, and the Raji cell line was provided by the laboratory of Professor Seung-Hwan Kim at Chungnam National University. CHO-K1 and Raji cells were maintained in RPMI 1640 supplemented with 10% FCS and 1% penicillin/streptomycin at 37°C under 5% CO₂. HEK293T cells were maintained in high-glucose DMEM supplemented with 10% FCS and 1% penicillin/streptomycin at 37°C under 5% CO₂. For cell culture, DMEM (Dulbecco's modified Eagle medium, 11995-065), RPMI 1640 (Roswell Park Memorial Institute medium, 11875-093), fetal bovine serum (FBS) (26140-079), penicillin-streptomycin (15140-122, Gibco, Life Technologies^{™}, Carlsbad, CA, USA) and 0.05% trypsin-EDTA solution (25300-062, Gibco, Life Technologies^{™}, Carlsbad, CA, USA) were used.

### Antibody cloning

The sequence of Rituximab was obtained from U.S. Patent Application Publication No. 2015-0141620 and the sequence of Obinutuzumab (GA101) was obtained from U.S. Patent No. 11,110,087. Nucleic acid sequences encoding the heavy chain and light chain of the anti-CD20 antibody were cloned into a pLVX vector and introduced into a lentivirus. The APEX2 sequence was obtained from Addgene (#49385, pcDNA3 Connexin43-GFP-APEX2).

### Lentivirus Production

293T cells were seeded in a 6-well plate at 8 × 10⁵ cells per well. After 6 hours, a total of 3 µg of target DNA and the lentiviral DNA clones pMD2G and psPAX2 were co-incubated with 100 µL of PBS and PEI (polyethylenimine) for 15 minutes, and then the mixture was added to the cells. After 6 hours of culture, the medium was replaced with complete DMEM, and the culture supernatant was collected 48 hours later and centrifuged at 1,000 rcf for 5 minutes at 4°C. The supernatant was filtered through a 0.45 µm PES syringe filter, transferred to a clean tube to remove cell debris, and stored at -70°C.

### Antibody Production

To produce anti-CD20 antibodies, 5 × 10⁴ CHO-K1 cells were seeded into each well of a 12-well plate. After 24 hours of culture, the cells were pretreated with polybrene (ant-pr-1, Invivogen, 10 mg/mL) 3 hours before infection. The cells were washed and treated with a mixture of lentivirus-containing culture medium and fresh culture medium at a ratio of 1:1, followed by incubation for 24 hours. The medium was replaced, and 10 µg/mL puromycin was added to select antibody-producing cells. After selection, the cells were expanded for antibody production. The obtained cells were cultured in OEX-cell CD CHO medium (10743011, Thermo Fisher) in a 30°C CO₂ incubator for 2 weeks. The culture medium was collected and centrifuged at 1,000 rcf for 3 minutes at 4°C, and the supernatant was transferred to a clean tube to remove mixed cells and then stored at 4°C. To produce the APEX2 fusion antibody, 3 × 10⁶ HEK293T cells were seeded in a 100pi culture dish. After 24 hours, 10 µg of APEX2 fusion antibody DNA was co-incubated with PEI in 200 µL of PBS for 15 minutes and then added to the cells. After 24 hours of culture, the medium was replaced with complete DMEM, and after 120 hours, the culture medium was collected, centrifuged at 1,000 rcf for 5 minutes at 4°C, and then stored at 4°C.

### Antibody Purification

Pierce Protein A Agarose (Thermo Fisher, QE218104) was added to the culture medium containing the antibody and reacted at 4°C for 24 hours. The medium was centrifuged at 2,000 rpm for 5 minutes. The beads were loaded onto an activated column and washed three times with cold PBS. The antibody was eluted from the beads using an elution buffer (75 µL of 0.1 M citric acid solution) and transferred to a tube containing 25 µL of 1 M Tris for neutralization. The eluted antibody was dialyzed twice against PBS for 6 hours and concentrated using Amicon^{®} Ultra-15 Centrifugal Filter Units (UFC900324, Merck, 3K). Total protein was quantified using a NanoDrop^{™} Lite spectrophotometer.

### SDS-PAGE Electrophoresis

After quantification, 3 µg of antibody was mixed with 6× Laemmli sample buffer and separated on a pre-cast 10% SDS-PAGE gel under both reducing and non-reducing conditions. The gel was stained with Coomassie Blue.

### Cell-Binding Assay

B-lymphoma Raji cells were resuspended in 50 µL of PBS together with anti-CD20 antibodies (obinutuzumab and rituximab), anti-CD20-APEX2 fusion antibodies (obinutuzumab-APEX2 and rituximab-APEX2) or control antibodies (trastuzumab and trastuzumab-APEX2) at concentrations of 7, 21, 35, and 70 nM, and incubated at 4°C for 30 minutes. After washing the cells with PBS, the cell pellet was resuspended in 50 µL of PBS together with an anti-human Ig Fc-specific FITC-conjugated secondary antibody diluted 1:500 (109-095-008, 1:200 dilution; Jackson Laboratories), and incubated at 4°C for 30 minutes. After washing twice with PBS, a total of 10,000 cells were counted using a flow cytometer (FACS LSR II SORP system, BD Biosciences, Franklin Lakes, NJ, USA) and analyzed using FlowJo software.

### Antibody-Dependent DCD Assay

For the DCD assay, Raji cells in each well were resuspended in 300 µL of RPMI complete medium and treated with designated concentrations of antibody (7, 21, 35, and 70 nM) or trastuzumab (7 nM) for 6 hours, followed by staining with 1 µL of 1 mg/mL PI at 37°C for 30 minutes. PE-positive cells were used as a readout of cell death, expressed as the percentage of fluorescently exposed cells among a total of 10,000 counted cells, and were counted using a flow cytometer (FACS LSR II SORP system, BD Biosciences, NJ, USA) and analyzed using FlowJo software.

### Optimization of APEX2 Proximity Labeling

Using 2 × 10⁵ B-cell lymphoma Raji cells, antibody concentration, biotin phenol concentration, H₂O₂ concentration, and incubation time were optimized for APEX2 proximity labeling. The reaction was performed in 50 µL of RPMI complete medium. For optimization of antibody concentration, each APEX2 fusion antibody at 1, 3, 5, and 10 µg/mL was treated together with 0.5 mM biotin phenol and H₂O₂. For optimization of incubation time, 5 µg/mL APEX2 fusion antibody was treated for 15 minutes, 30 minutes, and 1 hour. For biotinylation, 0, 0.25, 0.5, 2.5, and 5 mM biotin phenol or H₂O₂ was treated together with 0.5 mM H₂O₂ or biotin phenol, respectively, to confirm the efficiency of APEX2 according to the reagent. Biotinylated proteins were analyzed by Western blot.

### Protein Biotinylation Using APEX2 Fusion Antibody

Based on the optimization results of APEX2-dependent biotinylation, samples for HPLC-MS/MS were prepared. Raji cells at 1 × 10⁷ were prepared in a conical tube comprising 1 mL of RPMI complete medium, and each antibody (Obi-Tra-APEX2, Rit-APEX2, Tra-APEX2) was added to each tube and incubated at 37°C for 30 minutes. During incubation, 2× and 1× quenching solutions (0.5 mM MgCl₂, 1 mM CaCl₂, 5 mM Trolox, 10 mM sodium azide, 10 mM sodium ascorbate, PBS dissolved in distilled water) and a 9.8 mM H₂O₂ solution dissolved in PBS were prepared. The cells were centrifuged at 1,000 RCF for 5 minutes, and the supernatant was removed. The cells were washed twice and resuspended in 1 mL of pre-warmed RPMI complete medium and 0.5 mM biotin phenol. After 1 minute, 0.5 mM H₂O₂ solution was added to each tube and incubated for 1 minute, and then 1 mL of 2× quenching solution was immediately added to stop the biotinylation reaction. The biotinylated samples were centrifuged at 1,000 RCF for 5 minutes, the supernatant was removed, and the samples were gently mixed with 1× quenching solution. After additional centrifugation, the supernatant was discarded, and the cells were resuspended in 1 mL of RIPA buffer and quenching reagent, followed by incubation at 4°C for 20 minutes. The protein lysate was centrifuged at 18,000 RCF for 10 minutes at 4°C, and the supernatant containing biotinylated proteins was transferred to a new tube.

### Protein Biotinylation Using APEX2 Fusion Antibody

Based on the optimization results of APEX2-dependent biotinylation, samples for HPLC-MS/MS analysis were prepared. In a conical tube containing 1 mL of RPMI complete medium, 1 × 10⁷ Raji cells were prepared, and each antibody (Obi-Tra-APEX2, Rit-APEX2, Tra-APEX2) was added to each tube and incubated at 37°C for 30 minutes. During incubation, 2× and 1× quenching solutions (0.5 mM MgCl₂, 1 mM CaCl₂, 5 mM Trolox, 10 mM sodium azide, 10 mM sodium ascorbate, and PBS dissolved in distilled water) and a 9.8 mM H₂O₂ solution dissolved in PBS were prepared. The cells were centrifuged at 1,000 RCF for 5 minutes to remove the supernatant, washed twice, and resuspended in 1 mL of pre-warmed RPMI complete medium containing 0.5 mM biotin phenol. After 1 minute, 0.5 mM H₂O₂ solution was added to each tube and incubated for 1 minute. Immediately thereafter, 1 mL of 2× quenching solution was added to terminate the biotinylation reaction. The biotinylated samples were centrifuged at 1,000 RCF for 5 minutes, the supernatant was removed, and the samples were mixed with 1 mL of 1× quenching solution. After additional centrifugation, the supernatant was discarded, and the cells were resuspended in 1 mL of RIPA buffer and quenching reagent, followed by incubation at 4°C for 20 minutes. The protein lysate was centrifuged at 18,000 RCF for 10 minutes at 4°C, and the supernatant containing biotinylated proteins was carefully transferred to a new tube.

### Purification and Enrichment of Biotinylated Proteins

Biotinylated proteins were enriched using streptavidin agarose beads. The supernatant containing biotinylated proteins was incubated with the beads at 4°C with end-to-end rotation. After incubation for 2 hours, the tube was centrifuged at 500 RCF for 2 minutes and washed with 1 mL of wash buffer (RIPA buffer comprising 1 M KCl, 0.1 M Na₂CO₃, 2 M urea dissolved in 10 mM Tris-HCl). The beads were repeatedly washed with 1 mL of detergent-free RIPA buffer (10 mM Tris-HCl, pH 7.5, 150 mM NaCl, and 0.5 mM EDTA). After bead digestion for elution of biotinylated proteins was completed, the beads were incubated with 25 µL of elution buffer 1 (50 mM Tris-HCl, pH 7.5, 2 M urea, 5 µg/mL trypsin, and 1 mM DTT) in a Thermomixer at 400 RPM. After 30 minutes, the beads were centrifuged at 2,500 RCF for 2 minutes at 4°C, and the supernatant was transferred to a new tube. The beads were additionally resuspended in 25 µL of elution buffer 2 (50 mM Tris-HCl, pH 7.5, 2 M urea, and 5 mM iodoacetamide) and centrifuged twice at 2,500 g for 2 minutes at 4°C. Eluates 1 and 2 were mixed and incubated overnight in the dark at 32°C and 400 rpm in a Thermomixer to continue digestion. After 12 hours, 1 µL of trifluoroacetic acid was added to terminate the reaction.

### Western Blot and Silver Staining

Lysed samples obtained from the same number of cells were mixed with 6× Laemmli sample buffer, and 20 µL of each sample was loaded onto an acrylamide gel to separate proteins by size through electrophoresis. The gel was transferred to a nitrocellulose (NC) membrane and blocked with TBS-T containing 5% skim milk and 0.1% Tween 20. After incubation at room temperature for 1 hour, the membrane was reacted with a primary antibody for 1 hour and then washed three times with TBS-T for 10 minutes each. Thereafter, the membrane was reacted with a secondary antibody at room temperature for 1 hour. After washing three times with TBS-T for 10 minutes each, proteins were detected using an ECL solution. For silver staining, 20 µL of each sample mixed with 6× Laemmli sample buffer was separated on a pre-cast 10% SDS-PAGE gel under both reducing and non-reducing conditions. The gel was stained using a Pierce^{™} Silver Stain Kit (Thermo Fisher Scientific, Cat. No. 24612).

### HPLC-MS/MS

Protein samples digested from streptavidin agarose beads were separated and transferred to new tubes for HPLC-MS/MS (Bruker, timsTOF Pro 2). For quantitative analysis, the final protein target list was selected from the analyzed data according to the following strategy (FIG. 6): 1) the signal intensity of each detected protein was normalized to the total intensity value obtained from the analysis, and the list was prepared in descending order of intensity; 2) fold changes between the two anti-CD20 antibodies were calculated by dividing the normalized intensity of obinutuzumab-APEX2 by that of rituximab-APEX2; 3) proteins detected by more than two unique peptides were selected, and proteins overlapping with the control-APEX2 list were excluded; 4) proteins showing a change of more than 1.4-fold were selected; and 5) plasma membrane proteins were selected as final protein targets.

### Development of a Doxycycline-Induced shRNA-Dependent Knockdown System

A doxycycline-induced shRNA-dependent knockdown system was developed by cloning target shRNA antisense guides into a lentiviral vector. UTR-targeting antisense guide RNA sequences obtained from the SplashRNA website (http://splashrna.mskcc.org) were amplified by PCR. The amplified product was cloned into the XhoI/EcoRI sites of the LT3GEPIR plasmid having a pRRL backbone (Addgene, Plasmid #111177). For lentivirus production, the cloned vector was transfected into 8 × 10⁵ 293T cells together with psPAX2 and pMD2G. The obtained lentivirus was transduced into 5 × 10⁴ Raji cells, and the cells were selected using 5 µg/mL puromycin. After selection, 1 µg/mL doxycycline was added to 1 × 10⁶ Raji cells, and GFP-positive cells were isolated using a FACS Aria III (BD Biosciences, NJ, USA).

### Measurement of Lysosomal Membrane Permeabilization

For LMP measurement, 1 × 10⁵ Raji cells per well were prepared and resuspended in 100 µL of RPMI complete medium with 7 or 21 nM obinutuzumab or trastuzumab. After incubation for 4 hours, the cells were stained with LysoTracker^{™} Deep Red (Invitrogen^{™}, Cat. No. L12492) at 37°C for 30 minutes. Changes in the geometric mean of LysoTracker were calculated using a flow cytometer (FACS LSR II SORP system, BD Biosciences, NJ, USA) and analyzed using FlowJo software.

### RNA Extraction and qPCR for Quantification of Gene Expression

A total of 3 × 10⁶ shRNA-induced Raji cells were prepared by treating the cells with 0.1 µg/mL doxycycline for 3 days. The cells were resuspended in 500 µL of Trizol to isolate RNA and incubated at room temperature for 5 minutes to allow dissociation of nucleoprotein complexes. After incubation, 100 µL of chloroform was added, and the mixture was vigorously vortexed and incubated at room temperature. After 3 minutes, the sample was centrifuged at 13,000 RCF for 15 minutes at 4°C, and the colorless upper aqueous phase was transferred to a new tube. To precipitate RNA, 250 µL of isopropanol was added, and the mixture was incubated at room temperature for 10 minutes. The solution was centrifuged at 13,000 RCF for 10 minutes at 4°C, and the RNA pellet was washed twice with 500 µL of cold 75% ethanol. The RNA pellet was centrifuged at 7,500 RCF for 5 minutes and dried under vacuum for 5 minutes to remove residual ethanol. The RNA pellet was dissolved in 50 µL of distilled water and quantified using a NanoDrop^{™} Lite spectrophotometer.

The extracted RNA was reverse-transcribed using a SuperiorScript III cDNA Synthesis Kit (Enzynomics, Cat. No. EZ405S) and knockdown efficiency was measured using the obtained cDNA with a QuantStudio 3 Real-Time PCR Instrument (Applied Biosystems) and AccuPower^{®} 2× GreenStar qPCR Master Mix (Bioneer, K-6251). The PCR conditions were as follows: 95°C for 2 minutes for initial denaturation; and 95°C for 10 seconds and 58°C for 30 seconds for denaturation, annealing, extension, and measurement. Data were normalized by calculating ΔΔCT values between GAPDH and the target gene.

### FLAG Overexpression Cloning

For overexpression of target proteins, target genes were obtained from transcript variants (NCBI Gene, http://www.ncbi.nlm.nih.gov/gene/). Target genes were cloned into a pLVX vector to express target proteins having a 3× FLAG tag fused to the C-terminus. Lentivirus was produced in 293T cells and introduced into doxycycline-induced knockdown Raji cells. Selection was performed by treatment with 10 µg/mL blasticidin for 5 days, and protein overexpression was confirmed by Western blotting using an anti-FLAG antibody.

### Experimental Results

### Antibody-Dependent DCD Assay and LMP Assay

As a result of performing a cell death assay using in-house-prepared obinutuzumab and rituximab, it was confirmed that direct cell death (DCD) occurred only in response to obinutuzumab (FIG. 1a). In addition, as a result of an LMP (lysosome membrane permeabilization) assay, it was confirmed through LysoTracker^{™} staining that lysosomal disruption was also induced only by obinutuzumab (FIG. 1b).

### Validation of APEX2 Fusion Antibodies for Proximity Labeling

Fusion antibodies in which APEX2 was conjugated to each heavy chain of rituximab, obinutuzumab, and a negative control antibody (lacking CD20-binding ability due to an LC-N97A mutation) were cloned into a pLVX vector, produced by transient transfection in HEK293T cells, and confirmed by SDS-PAGE (FIG. 2a). As a result of performing a CD20 binding assay by flow cytometry using the produced APEX2 fusion antibodies and the negative control antibody, it was confirmed that the APEX2 fusion antibodies had lower binding ability than the wild-type antibodies, whereas the negative control antibody exhibited nonspecific binding (FIG. 2b). To improve these results, a GGGS × 4 linker was introduced between the C-terminus of the antibody heavy chain and APEX2 (FIG. 2d), and it was observed that the resulting antibody exhibited CD20-binding ability equivalent to that of the wild-type antibody under non-reducing conditions (FIG. 2c).

### Validation of Second-Generation APEX2 Fusion Antibodies for Proximity Labeling

Fusion antibodies were prepared in which APEX2 was linked via a GGGGS × 4 linker to each heavy chain of chimeric antibodies comprising the heavy chain of obinutuzumab, rituximab and trastuzumab and the light chain of obinutuzumab (FIG. 3a). Expression and purification of each antibody were confirmed by SDS staining under non-reducing and reducing conditions (FIG. 3b). Binding assays (FIG. 3c) and cell death assays (FIGS. 3d and 3e) were performed using Raji cells treated with 35 nM anti-CD20 antibodies (obinutuzumab, rituximab), anti-CD20-APEX2 fusion antibodies (obinutuzumab-APEX2 and rituximab-APEX2) and control antibodies (trastuzumab and trastuzumab-APEX2). As a result, it was confirmed that trastuzumab (Tra) and trastuzumab-obinutuzumab-linker-APEX2 (OTA) showed reduced CD20-binding ability and reduced cell death-inducing ability.

### Optimization of Biotinylation Using APEX2 Fusion Antibodies

As a result of confirming protein biotinylation efficiency according to the concentrations of biotin phenol and H₂O₂, 5 mM biotin phenol and 2 mM H₂O₂ were selected as optimal concentrations, respectively (FIG. 4a). Using these selected concentrations, the antibody concentration (FIG. 4b) and antibody incubation time (FIG. 4c) showing the highest biotinylation efficiency were selected. In addition, each biotinylated antibody (Obi-Tra-APEX2, Rit-APEX2, Tra-APEX2) was confirmed by Western blotting (FIG. 5a) and silver staining (FIG. 5b) performed according to the above-described methods.

### Selection of Target Proteins Based on HPLC-MS/MS Analysis Results

The list of target proteins or genes predicted to be involved in DCD which were selected through HPLC-MS/MS analysis according to the above-described criteria (FIG. 6) is shown in FIG. 7a. The list shows fold change values obtained by dividing intensity values in protein samples obtained using obinutuzumab-linker-APEX2 by intensity values obtained using rituximab-linker-APEX2. In addition, as a result of analyzing the effect of proteins knocked down by doxycycline-induced shRNA on cell death using an LMP assay, no difference was observed in Raji wild-type cells, the shLuc control, or the positive control shCD20 doxycycline (-) group, whereas obinutuzumab-induced cell death was reduced only in the shCD20 doxycycline (+) group. Thus, it was confirmed that the doxycycline-induced shRNA-dependent knockdown system functioned properly (FIG. 8b). In addition, LMP induced by obinutuzumab in the doxycycline-induced shRNA-dependent knockdown system was confirmed by LysoTracker Deep Red staining (FIG. 9a), and knockdown was verified by qPCR (FIG. 9b).

### Selection of DCD-Associated Proteins

A DCD (direct cell death) assay was performed using candidate groups that showed significant results in the preceding LMP assay (FIG. 10a). For MPZL1 and HLA-E, which were the two target proteins having the greatest effect on cell death compared to the control group, the effect on cell death was confirmed according to the duration of doxycycline treatment (6, 9, 12 and 15 days) (FIG. 10b). To induce knockdown by doxycycline-induced shRNA, cells were treated with 0.1 µg/mL doxycycline on days 1 and 4 at 2 × 10⁵ cells in 3 mL. Each 1 × 10⁵ cells were treated with 7 nM obinutuzumab antibody for 6 hours, followed by PI staining for 30 minutes. Thereafter, the percentage of PI-positive cells was calculated using a flow cytometer, and fold change was calculated by dividing each PI-positive percentage by the value of cells not treated with doxycycline (-).

### Confirmation of Effects by Overexpression of Candidate Proteins

To confirm the effects of the two selected candidate proteins on cell death, a CRISPRa overexpression system was applied. To construct a CRISPR activation system and overexpress target genes, sequences corresponding to guide RNAs were designed and synthesized using the SplashRNA website (http://splashrna.mskcc.org/) and cloned into a lentiviral vector. MCP, P65, and HSF1 transcription factors corresponding to SAM were also prepared by cloning into lentiviral vectors, and each gene vector was transfected into 293T cells. Thereafter, overexpressing cells were prepared by transducing Raji cells with SAM and dCas9 lentiviruses. After stable cell lines were established by selection with 10 µg/mL blasticidin, overexpression was confirmed by qPCR after RNA extraction and cDNA synthesis (FIG. 12a), and overexpression of the target protein was verified by Western blotting using an anti-MPZL1 antibody. Specifically, 1 × 10⁶ knockdown and overexpressing cells on day 6 were lysed in 500 µL of RIPA buffer, and 30 µL aliquots were subjected to electrophoresis. Detection was performed using an anti-human MPZL1 mouse antibody diluted 1:1000 as a primary antibody and an anti-mouse HRP antibody diluted 1:5000 as a secondary antibody (FIG. 12b). As a result of performing a cell death assay using the established MPZL1-overexpressing Raji cells, it was confirmed that cell death was significantly increased by MPZL1 overexpression (FIG. 12c).

### Effect of Concanavalin A, a Ligand of MPZL1, on Cell Death

To observe changes in the degree of cell death, 1 × 10⁵ Raji cells knocked down by doxycycline-induced shRNA were treated with 10 µg/mL concanavalin A, a ligand of MPZL1, for 6 hours. After adding 1 µL of 1 mg/mL PI and staining at 37°C for 30 minutes, PI-positive cells were counted and expressed as a percentage of the total 10,000 cells. As a result of counting using a flow cytometer (FACS LSR II SORP system, BD Biosciences, Franklin Lakes, NJ, USA) and analyzing with FlowJo software, it was confirmed that cell death was significantly reduced in MPZL1-knockdown cells (FIG. 13a). In addition, as a result of confirming DCD (direct cell death) according to the concentrations of concanavalin A and anti-CD20 antibodies (rituximab, obinutuzumab), it was observed that a synergistic cell death effect was exerted when cells were treated with concanavalin A (0, 0.3, 1, 3 µg/mL) and each antibody (0, 0.3, 1 µg/mL) (FIG. 13b).

### Preparation of Concanavalin A Fusion Antibody

Since it was confirmed that cell death was increased upon co-treatment with concanavalin A and an anti-CD20 antibody, a fusion antibody in which concanavalin A was conjugated to the Fc region of an anti-CD20 antibody through the GGGGS × 4 linker used for APEX2 fusion was prepared (FIGS. 14a and 14b), and the purified antibody was confirmed by SDS-PAGE (FIG. 14c). For the fusion antibody, 3.5 × 10⁶ HEK293T cells were seeded in a 100pi culture dish, and transient transfection was performed using 10 µg of target DNA at a ratio of 1 µg DNA:3 µL PEI. After 6 hours, the medium was replaced, and 24-hour rescue was performed. The medium was then replaced with 10 mL of 293 Freestyle medium, followed by culture for 6 days. After all supernatant was discarded, the culture medium containing the antibody was reacted with Agarose A beads (Pierce Protein A Agarose, Thermo Fisher, QE218104) at 4°C for 24 hours. Thereafter, the mixture was centrifuged at 2,000 rpm for 5 minutes, the beads were collected onto an activated column, and the beads were washed three times with cold PBS. The antibody was eluted from the beads using an elution solution (75 µL of 0.1 M citric acid), and 25 µL of 1 M Tris was added for neutralization. The eluted antibody was dialyzed twice against PBS for 6 hours and then concentrated using Amicon^{®} Ultra-15 centrifugal filter units (UFC900324, Merck, 3K). The total protein amount was measured using a NanoDrop^{™} Lite spectrophotometer.

### Confirmation of Effects of Concanavalin A Fusion Antibody

To compare the effects of rituximab-concanavalin A (Rit-ConA) and rituximab, 1 × 10⁵ Raji cells were treated with 2.1 nM or 7 nM antibody for 6 hours, and the cell death rate was measured by PI staining. As a result, it was confirmed that Rit-ConA showed a markedly superior cell death-inducing effect (FIG. 15a). In addition, comparison of the LMP effect also showed that the concanavalin A fusion antibody induced cell death more effectively than the wild-type antibody (FIG. 15b). It was also confirmed that higher cytotoxicity was exerted by the antibody fused into a single molecule than by simple co-administration of concanavalin A and an anti-CD20 antibody (FIG. 15c). Since two monomers of concanavalin A are conjugated to the antibody, the analysis was performed such that the antibody:concanavalin A ratio was 1:2 during co-administration.

Finally, to confirm the CD20 binding ability of each antibody, 2 × 10⁶ Raji cells were resuspended in 50 µL of PBS containing 21 nM anti-CD20 antibody, i.e., obinutuzumab or rituximab, anti-CD20-ConA fusion antibody, or control antibody, i.e., trastuzumab or trastuzumab-ConA, and reacted at 4°C for 30 minutes. After washing the cells with PBS, the cells were resuspended in 50 µL of PBS containing an anti-human Ig Fc-specific FITC-conjugated secondary antibody (109-095-008, 1:200 dilution; Jackson Laboratories) at a 1:500 dilution ratio and reacted at 4°C for 30 minutes. After washing twice with PBS, a total of 10,000 cells were detected using a flow cytometer (FACS LSR II SORP system, BD Biosciences, Franklin Lakes, NJ, USA) and analyzed using FlowJo software. As a result of the analysis, the binding ability of the ConA fusion antibody was slightly reduced compared to that of the unconjugated wild-type antibody (FIG. 15d).

Additionally, after 1 × 10⁵ Raji cells were treated with various concentrations of each antibody, PI staining and LysoTracker Green staining were performed 6 hours later. As a result, it was also confirmed that the cell death-inducing effect of the ConA fusion antibody was superior to that of the control group (FIG. 16a), and similar results were observed in experiments using 1 × 10⁵ Ramos cells (FIG. 16b).

**[Table 1]**

| **Amino acid sequences of the antibodies and concanavalin A monomers used in the present invention** | | |
|---|---|---|
| Sequence No. | Peptide | Sequence |
| 1 | Obi-HCDR1 | YSWIN |
| 2 | Obi-HCDR2 | RIFPGDGDTDYNGKFK |
| 3 | Obi-HCDR3 | NVFDGYWLVY |
| 4 | Obi-LCDR1 | RSSKSLLHSNGITYLY |
| 5 | Obi-LCDR2 | QMSNLVS |
| 6 | Obi-LCDR3 | AQNLELPYT |
| 7 | Obi-heavy chain variable region | QVQLVQSGAEVKKPGSSVKVSCKASGYAFSYSWINWVRQ APGQGLEWMGRIFPGDGDTDYNGKFKGRVTITADKSTST AYMELSSLRSEDTAVYYCARNVFDGYWLVYWGQGTLVT VSS |
| 8 | Obi-light chain variable region | DIVMTQTPLSLPVTPGEPASISCRSSKSLLHSNGITYLYWYL QKPGQSPQLLIYQMSNLVSGVPDRFSGSGSGTDFTLKISRV EAEDVGVYYCAQNLELPYTFGGGTKVEIK |
| 9 | Rit-HCDR1 | SYNMH |
| 10 | Rit-HCDR2 | AIYPGNGDTSYNQKFKG |
| 11 | Rit-HCDR3 | STYYGGDWYFNV |
| 12 | Rit-LCDR1 | RASSSVSYIH |
| 13 | Rit-LCDR2 | ATSNLAS |
| 14 | Rit-LCDR3 | QQWTSNPPT |
| 15 | Rit-heavy chain variable region | QVQLQQPGAELVKPGASVKMSCKASGYTFTSYNMHWVK QTPGRGLEWIGAIYPGNGDTSYNQKFKGKATLTADKSSST AYMQLSSLTSEDSAVYYCARSTYYGGDWYFNVWGAGTT VTVASA |
| 16 | Rit-light chain variable region | |
| 17 | Con A monomer | MADTIVAVELDTYPNTDIGDPSYPHIGIDIKSVRSKKTAKW NMQNGKVGTAHIIYNSVDKRLSAVVSYPNADSATVSYDV DLDNVLPEWVRVGLSASTGLYKETNTILSWSFTSKLKSNS THETNALHFMFNQFSKDQKDLILQGDATTGTDGNLELTRV SSNGSPQGSSVGRALFYAPVHIWESSAVVASFEATFTFLIK SPDSHPADGIAFFISNIDSSIPSGSTGRLLGLFPDAN |

Having described specific embodiment of the present invention in detail above, it is to be understood that variants and modifications thereof falling within the spirit of the invention may become apparent to those skilled in this art, and the scope of this invention is to be determined by appended claims and their equivalents.

## Claims

1. A recombinant polypeptide that specifically binds to B-lymphocytes, comprising:
(a) an antibody or an antigen-binding fragment thereof that specifically binds to a B-lymphocyte antigen; and
(b) a concanavalin A protein or a functional fragment thereof bound to the antibody or antigen-binding fragment thereof of (a).

2. The recombinant polypeptide of claim 1, wherein the B-lymphocyte specific antigen is one or more antigens selected from the group consisting of CD19, CD20, CD30 and CD79b.

3. The recombinant polypeptide of claim 1, wherein the concanavalin A protein or a functional fragment thereof binds to the C-terminus of the antibody or the antigen-binding fragment thereof.

4. The recombinant polypeptide of claim 3, wherein the concanavalin A protein or a functional fragment thereof is linked to the C-terminus of the antibody or the antigen-binding fragment thereof via a linker.

5. The recombinant polypeptide of claim 4, wherein the linker is a flexible peptide linker represented by (GGGGS)n (n is an integer from 1 to 5).

6. The recombinant polypeptide of claim 1, wherein the functional fragment of concanavalin A is a dimer comprising two monomer subunits that constitute a concanavalin A tetramer.

7. The recombinant polypeptide of claim 6, wherein the two monomer subunits of the concanavalin A bind to the C-terminus of each heavy chain of the antibody that specifically binds to the B-lymphocyte antigen.

8. The recombinant polypeptide of claim 2, wherein the B-lymphocyte specific antigen is CD20.

9. The recombinant polypeptide of claim 8, wherein the antibody that specifically binds to the B-lymphocyte specific antigen is obinutuzumab or rituximab.

10. A nucleic acid molecule encoding the recombinant polypeptide of claim 1.

11. A gene delivery system comprising the nucleic acid molecule of claim 10.

12. A host cell transformed with the gene delivery system of claim 11.

13. A composition for preventing or treating B-cell lymphoma comprising, the recombinant polypeptide of claim 1, the nucleic acid molecule of claim 10 or the gene delivery system of claim 11, as an active ingredient.

14. The composition of claim 13, wherein the B-cell lymphoma is selected from the group consisting of diffuse large B-cell lymphoma (DLBCL), follicular lymphoma, marginal zone B-cell lymphoma (MZL), small lymphocytic lymphoma (SLL) and mantle cell lymphoma (MCL).

15. A composition for preventing or treating B-cell lymphoma, comprising:
(a) an antibody or antigen-binding fragment thereof that specifically binds to a B-lymphocyte antigen, or a nucleic acid molecule encoding the same; and
(b) a myelin protein zero like 1 (MPZL1) protein, or a nucleic acid molecule encoding the same.
